# EUROPEAN PATENT SPECIFICATION

(11) **EP 1 433 415 B1**
(45) Date of publication and mention of the grant of the patent: **13.02.2008**
(21) Application number: 04090116.7
(22) Date of filing: 30.03.2001
(51) Int. Cl.: A61B 3/04

(54) **Eyeglass frame**
Brillenfassung
Monture de lunettes

(30) Priority: 30.03.2000 JP 2000093051; 16.03.2001 JP 2001076342
(43) Date of publication of application: 30.06.2004
(62) Divisional of application: 01250112.8
(73) Proprietor: Kabushiki Kaisha TOPCON, Tokyo 174-0052 (JP)
(72) Inventor: Kato, Takeyuki, c/o Kabushiki Kaisha TOPCON, Tokyo 174-0052 (JP); Fukuma, Yasufumi;c/o Kabushiki Kaisha TOPCON, Tokyo 174-0052 (JP)
(74) Representative: Pfenning, Meinig & Partner GbR

(56) References cited:
- DE-A- 4 117 754
- GB-A- 1 278 429
- US-A- 3 305 294
- US-A- 3 588 235
- US-A- 3 758 201
- US-A- 4 798 457
- US-A- 5 104 214

## Description

### DESCRIPTION OF THE PRIOR ART

Generally, the refraction property of long-sightedness, short-sightedness, or astigmatism of the tested eye is measured using an objective refract meter or a subjective eye test apparatus such as a visual tester, and a prescription value such as S (spherical degree), C (cylindrical degree), A (axis angle of the cylindrical axis) of the eyeglasses is prepared from the refraction property. Such obtained prescription value can be referred to as the perfect prescription value because it is prepared to perfectly correct the long-sightedness, the shortsightedness and the astigmatism.

However, until now, when an test subject which cannot see well due to the long-sightedness, the short-sightedness or the astigmatism wears the eyeglasses prepared based on the perfect prescription value, the test subject feels dizziness or headache, or may feel eye fatigue.

For example, in case where the vision 0.2 of the test subject becomes 1.2 by the perfect prescription value, the diopter of the perfect prescription value of eyeglasses having the vision 1.2 is -4.5. However, when the vision 0.2 is suddenly corrected to 1.2, the test subject often feels dizziness or headache, or may feel eye fatigue because the correction width is large.

Accordingly, conventionally, the value lower than the perfect prescription value obtained by measuring the S, C, A values of the actually prepared eyeglasses has been used as the eyeglass prescription value, thereby the dizziness, the headache or the eye fatigue has been suppressed if at all possible. Here, when the vision 0.2 of the test subject becomes 1.2 by the perfect prescription value, for example, the corrected vision may be 0.8 lower than the vision 1.2 by the perfect prescription value.

However, in case of preparing the above-mentioned eyeglass prescription value, after the perfect prescription value is prepared by a refract meter or a vision tester, a wearing test is performed by a lens exchange method based on the prescription value lower than the perfect prescription value, and the final confirmation is performed.

In this lens exchange method, the reference lens of the suitable diopter (one of the exchange lenses) is provided in the eyeglass frame for test such as a trial frame, and a plurality of exchange lenses for increasing and decreasing the diopter of the reference lens little by little are prepared, and the exchange lenses are provided in the trial frame one by one, and the final well-seen state is selected, asking the test subject about the seen state. For example, in case where the vision 0.2 of the test subject becomes 1.2 by the perfect prescription value, when prescribing the eyeglasses for obtaining the final vision, the reference lens of -4.5D is set to the trial frame, and at the same time, a plurality of exchange lenses of - 0.25D are prepared, the lenses are provided in the trial frame one by one, and the final well-seen state is selected, asking the test subject about the seen state. And, in this state, when the person wears the trial frame for several tens of minutes, if the person does not feel inconvenience, the total diopter of the lens by the reference lens and the exchange lenses at this time becomes the eyeglass prescription value.

Generally, the eyeglass prescription value is taken a memo, and the process such as the operation for inputting the prescription value memo to a personal computer through a keyboard is performed. Thus, conventionally, the final eyeglass prescription value input by the trial frame was complicated.

US 3 588 235 describes an apparatus for measuring spherical refraction error and stigmatism. A combination lens system comprises a spherical fluid lens and a cylindrical fluid lens, the two lenses being aligned for concurrent use. The dioptric value of each lens is varied by introducing and removing liquid by means of a controlled bellows pump.

### SUMMARY OF THE INVENTION

Thus, the object of the present invention is to provide an eyeglass frame and an eye test system which comprises means for variably adjusting the spherical degree, the cylindrical degree and the cylindrical axis of the eyeglass frame for test in order to obtain the final prescription data of the eyeglasses for a test person.

The object of the present invention is accomplished by the features of claim 1 and claim 8.

### BRIEF DESCRIPTION OF THE ATTACHED DRAWINGS

FIG. 1(a) is a front view of the eye test system according to the present invention, FIG. 1(b) is a plan view of FIG. 1(a), and FIG. 1(c) illustrates the state that the lens receiving drawer in FIG. 1(a) is taken out;
FIG. 2 is a control circuit diagram of the eye test system shown in FIG. 1;
FIG. 3 is a perspective view showing a trial frame used in the eye test system in FIG. 1;
FIG. 4 is a horizontal cross-sectional view of the head frame in the trial frame in FIG. 3;
FIG. 5 is a cross-sectional view along the line A-A of FIG. 4;
FIG. 6 illustrates a part of the trial frame in FIG. 3;
FIG. 7 is a cross-sectional along the line B-B in FIG. 6;
FIG. 8 is a rear view of the lens attaching frame shown in FIG. 3;
FIG. 9 illustrates the attaching portion of the plate spring in FIG. 8;
FIG. 10(a) illustrates the lens supporting protrusion in FIGS. 3 and 6, FIG. 10(b) illustrates the tail of the plate spring attaching portion in FIGS. 3 and 6;
FIG. 11 illustrates a portion of the rear surface of the lens attaching frame shown in FIG. 3;
FIG. 12 is a cross-sectional view along the line C-C in FIG. 11;
FIG. 13 is a cross-sectional view along the line D-D in FIG. 12;
FIG. 14 is a rear view of the rotary lens attaching frame shown in FIG. 3;
FIG. 15 is a partially enlarged perspective view showing the lower side of the lens attaching frame in FIG. 3;
FIG. 16 is a partially enlarged explaining view of the lens receiving drawer in FIG. 1(c)

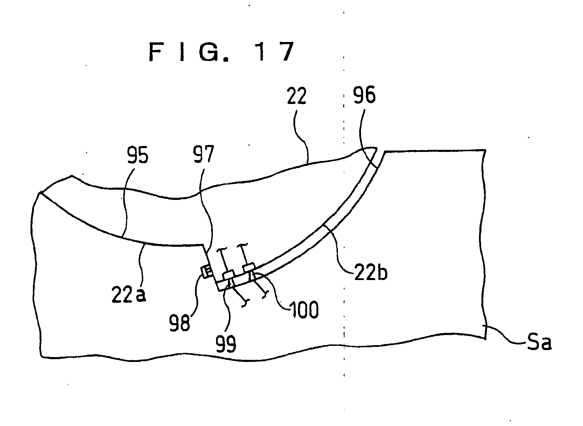

FIG. 17 is a cross-sectional view along the line E-E in FIG. 16;
FIG. 18 is a cross-sectional view along the line F-F in FIG. 3;
FIG. 19 is a cross-sectional view along the line G-G in FIG. 18;
FIG. 20 is a cross-sectional view along the line H-H in FIG. 18;
FIG. 21 is a cross-sectional view along the line I-I in FIG. 18;
FIG. 22 is a partially enlarged illustrating view of FIG. 18;
FIG. 23 illustrates FIG. 22 viewed from the arrow J direction;
FIG. 24(a) is a cross-sectional view showing the temple attaching portion shown in FIG. 3, FIG. 24(b) illustrates FIG. 24(a) viewed from the arrow K direction, and FIG. 24(c) illustrates the temple length detecting means provided in the first arm in FIG. 24(a);
FIG. 25(a) is a plane view of the exchange lens used in the eye test system of the present invention, and FIG. 25(b) is a bottom view of FIG. 25(a);
FIG. 26 illustrates an example of the display screen of the liquid crystal display device in FIG. 1;
FIG. 27 illustrates a modified example of the trial frame used in the present invention;
FIG. 28 is a partially cross-sectional view showing the information transmitting structure of the trial frame in FIG. 27;
FIG. 29 illustrates another example of the exchange lens used in the trial frame;
FIG. 30 shows an example of the information detecting structure of the trial frame using the exchange lens in FIG. 29;
FIG. 31 illustrates another example of the information detecting structure of the trial frame of the present invention;
FIG. 32(a) is a cross-sectional view showing a modified example for detecting the position information of the forward and backward position of the nosepiece, and FIG. 32(b) is a perspective view of the main part showing an example of scale used in adjusting the forward and backward position of the nosepiece in FIG. 32(a);
FIG. 33 is a perspective view of the trial frame showing another example for adjusting the movement of the lens attaching frame shown in FIG. 3;
FIG. 34 is a side view showing an example of another trial frame of the present invention;
FIG. 35 illustrates an example in which the trial frame in FIG. 34 is used;
FIG. 36 is a side view showing an example of another trial frame of the present invention;
FIG. 37 is a side view showing an example of another eyeglass frame for test according to the present invention;
FIG. 38 is a front view of the eyeglass frame for test in FIG. 37;
FIG. 39 is a cross-sectional view along the line K-K of the lens attaching portion in FIG. 38;
FIG. 40 is a horizontal cross-sectional view along the line L-L of a part of the variable focus lens portion in FIG. 39;
FIG. 41 is a vertical cross-sectional view along the line M-M of the variable focus lens in FIG. 39;
FIG. 42 illustrates the driving system of the Vcc lens in FIG. 39;
FIG. 43 is a perspective view for explaining the variable focus lens and the Vcc lens in FIG. 39; and
FIG. 44 is a control circuit diagram of the eyeglass frame for test in FIGS. 37-43.

### DESCRIPTION OF THE PREFERRED EMBODIMENT

The embodiments of the present invention will be explained with reference to the accompanying drawings.

### [Composition]

### <System scheme>

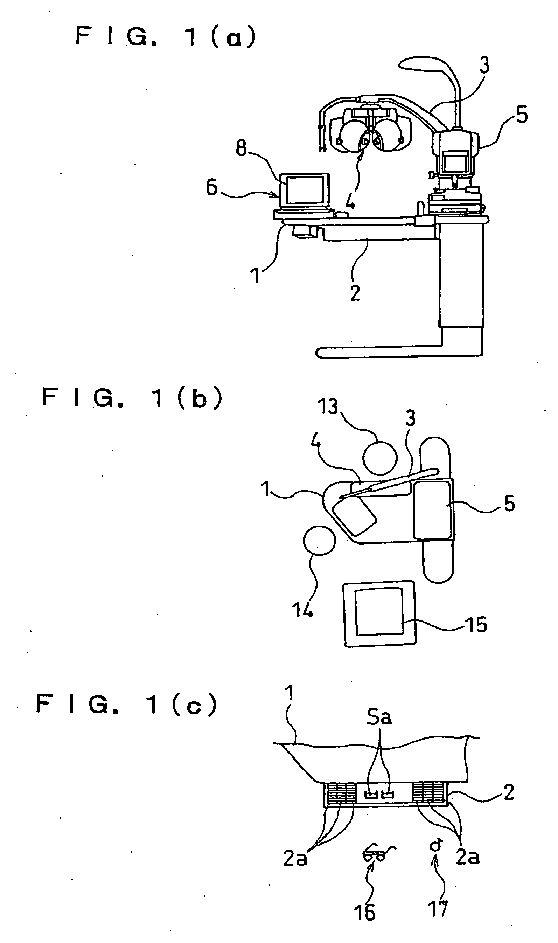

FIG. 1(a) is a front view of an eye test system according to the present invention, FIG. 1(b) is a plane view of FIG. 1(a), and FIG. 1(c) illustrates a part of FIG. 1(a). In FIG. 1, 1 is an eye test table of which the vertical movement can be adjusted by an electric motor (not shown), 2 is a lens receiving drawer of the eye test table 1, 3 is a supporting arm attached on the eye inspection table 1 to be rotatable horizontally and movable upwardly and downwardly, 4 is a vision tester suspended on the supporting arm 3 (subjective eye test unit, that is, subjective eye test apparatus), 5 is an autorefractometer provided on the eye test table 1 to be movable leftward and rightward, and 6 is a personal computer laid on the eye test table 1.

### <Personal computer 6>

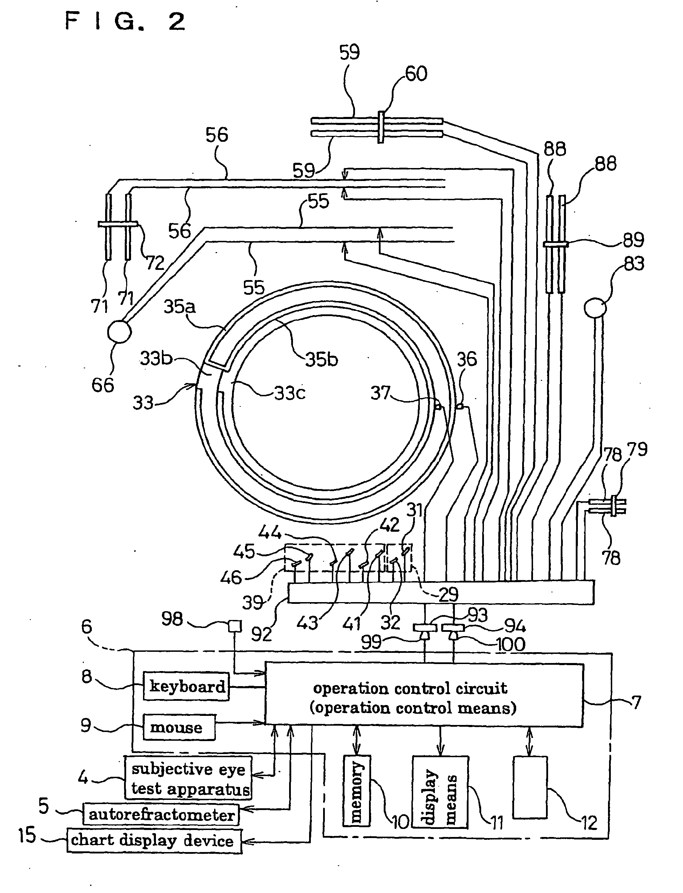

As shown in FIG. 2, the personal computer 6 has an operation control circuit (operation control means) 7, a keyboard (data inputting means) 8, a mouse (an operation means of screen and data, etc.) 9, a memory 10, a liquid crystal display device (display device, that is, display means) 11, and an information record reproducing device 12 such as hard disk, optical magnetic disk or DVD. The keyboard 8 is used in the data input or the screen operation of the liquid crystal display device 11, and the mouse 9 is used in the screen operation of the liquid crystal display device 11 or the data input. Also, the eye test data from the vision tester 4 and the autorefractometer 5 are inputted to the operation control circuit 7. And, the operation control circuit 7 allows the memory 10 to store the inputted eye test data, allows the liquid crystal display device 11 to display the eye test data stored in the memory 10, and at the same time, allows the information record reproducing device 12 to record the eye test data.

### <Chart display device>

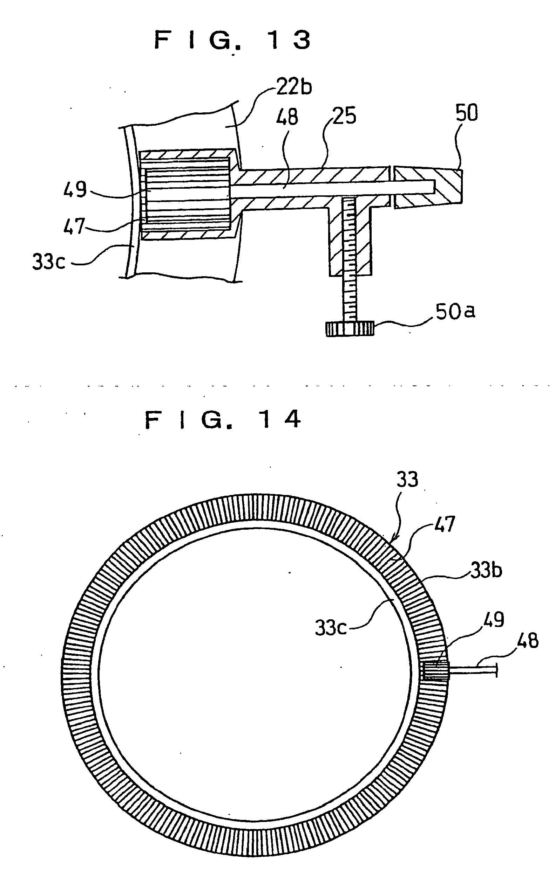

In addition, in FIG. 1(b), 13 is a test subject, 14 is a testing person, and 15 is a chart display device for displaying an eye test chart. The chart display device 15 is arranged with the eye test table sandwiched therebetween, and simultaneously, converts display of the eye test chart such as a Landolt ring chart, a hiragana chart, a radial astigmatism chart by the operation of the personal computer 6 to allow the test subject 13 to view the displayed eye test chart.

### <Trial eye test apparatus>

In FIG. 1(c), 2a is a lens receiving portion provided in the lens receiving drawer 2, 2b is a frame receiving portion provided in the lens receiving drawer 2, 16 is a trial frame (eyeglass frame for test) used in the lens exchange method, L is an exchange lens attached and detached to the trial frame 16. The frame receiving portion 2b has sliding members sa, sa which are arranged at intervals leftward and rightward and are movable leftward and rightward, and frame inserting concave portions sb, sb provided in the sliding members sa, sa. The frame inserting concave portion sb has a small-diameter concave portion 95 and a large-diameter concave portion 96, and a step surface 97 is provided as a stopper between the small-diameter concave portion 95 and the large-diameter concave portion 96.

The exchange lens L has a ring-shaped lens frame La, a lens Lb in the lens frame La, a handle Lc provided in the lens frame La. And, the lens frame La is provided with the reference line (reference mark) Ld as the positioning line (positioning mark).

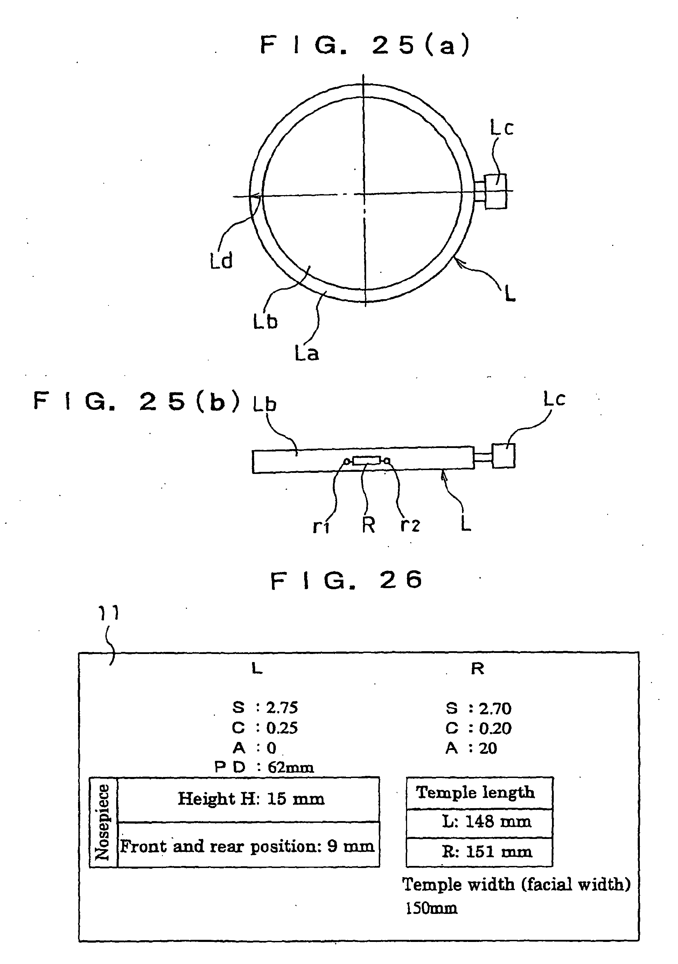

As shown in FIG. 25(b), the periphery surface of the exchange lens L is provided with a resistor R for detecting the refraction degrees of the cylindrical axis and the spherical lens, and terminals r1, r2 of the resistor R. In case where the resistor R is not less than a predetermined value, the refraction degree of the cylindrical lens can be detected by the difference between the resistor values not less than the predetermined value, and in case where the resistor R is less than the predetermined value, the refraction degree of the spherical lens can be detected by the difference between the resistor values less than the predetermined value.

### <Trial frame>

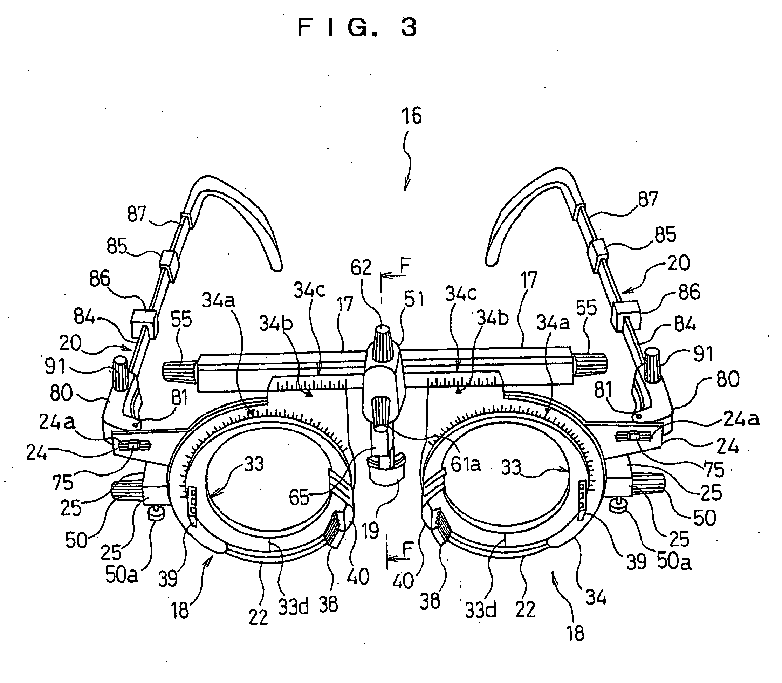

As shown in FIG. 3, the trial frame 16 has a head frame 17 extended leftward and rightward, lens attaching frames (movable portion) 18, 18 respectively provided in the left and right lower sides of the head frame 17, a nosepiece 19 located between both the lens attached frames 18, 18, and temples (movable portion) 20, 20 provided in the left and right ends of the head frame 17.

### (Lens attaching frame)

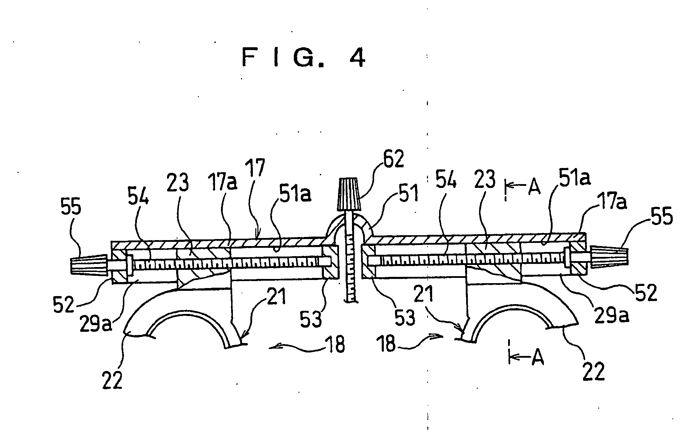

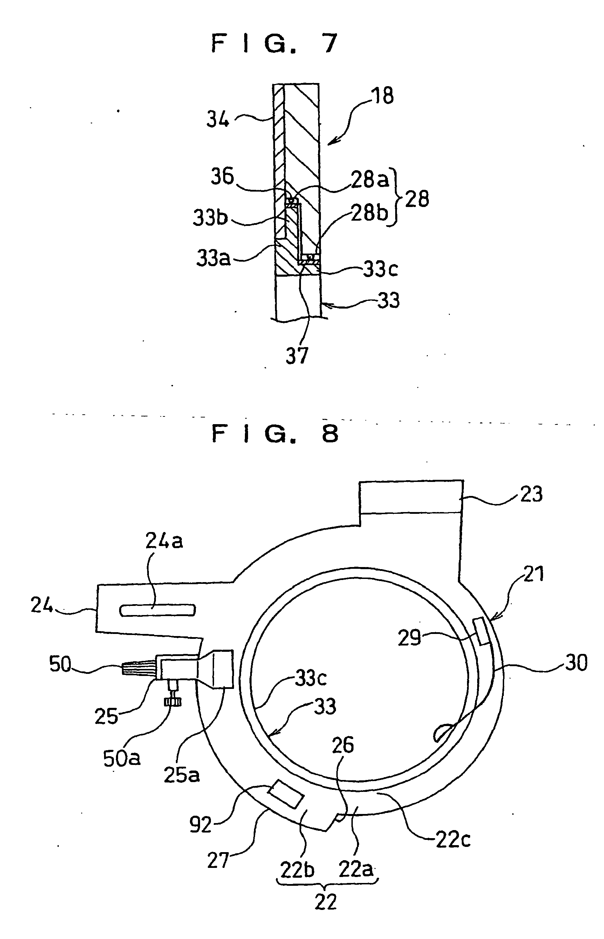

As shown in FIG. 8, the lens attaching frame 18 has a plate-shaped attaching frame body (movable lens attaching frame) 21. As shown in FIG. 8, the attaching frame body 21 has a ring-shaped portion (ring portion) 22, a supporting portion 23 protruded from the upper end portion of the ring-shaped portion 22, a temple attaching portion 24 protruded from the upper portion of the side surface of the ring-shaped portion 22 toward the side, and a bearing portion 25 located at the lower side of the temple attaching portion 24 and protruded from the ring-shaped portion 22 (refer to FIGS. 3 and 4).

The ring-shaped portion 22 has a small-diameter portion 22a and a large-diameter portion 22b. 26 is a step surface between the small-diameter portion 22a and the large-diameter portion 22b, and 27 is a periphery surface of the large-diameter portion 22b. The temple attaching portion 24 is provided with a guide slit 24a extended leftward and rightward, and the bearing portion 25 is continuously provided with a receiving portion 25a.

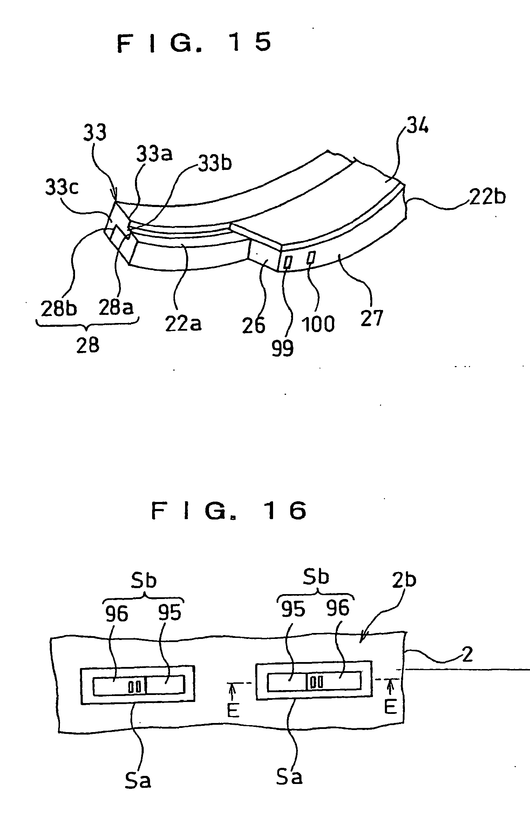

A fitting hole 28 is formed in the center of the ring-shaped portion 22. As shown in FIGS. 7 and 15, the fitting hole 28 comprises a large-diameter hole portion 28a of the front side and a small-diameter hole portion 28b of the rear side.

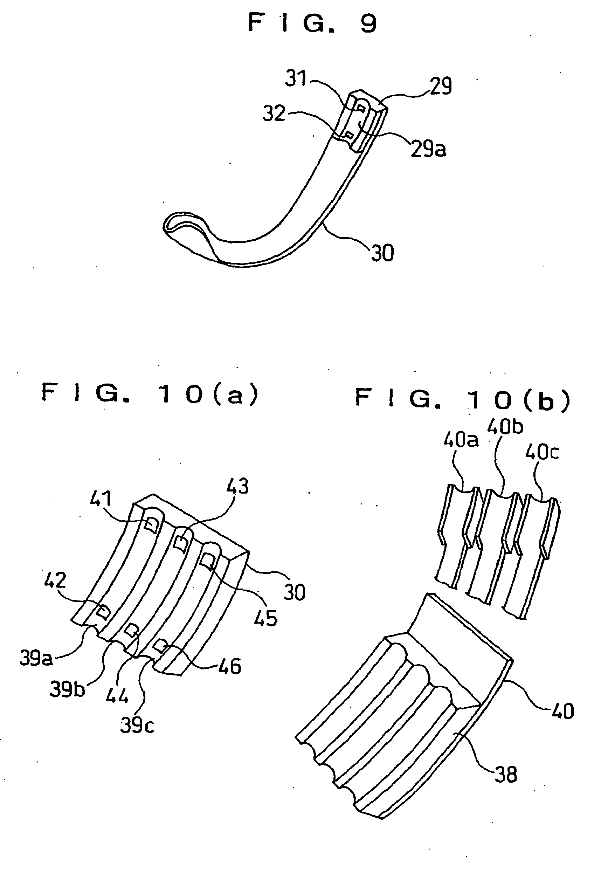

As shown in FIG. 8, a lens supporting protrusion 29 is protruded from the rear surface of the attaching frame body 21, and an end portion of a lens supporting plate spring 30 is fixed to the lens supporting protrusion 29. As shown in FIG. 9, the lens supporting protrusion 29 is provided with a lens coupling groove 29a, and the lens coupling groove 29a is provided with detecting terminals (detecting means) 31, 32 as an information detecting portion.

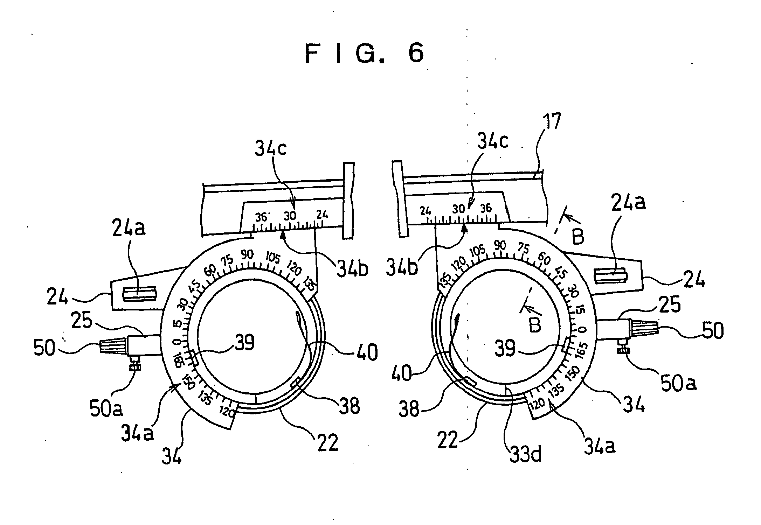

In addition, as shown in FIGS. 6 to 8, the lens attaching frame 18 has a ring-shaped rotary lens attaching frame 33 rotatably fitted to the fitting hole 28. The rotary lens attaching frame (the movable portion) 33 has a middle-diameter portion 33a, a large-diameter flange 33b protruded from the center of the thickness direction of the plate and rotatably fitted to the large-diameter hole portion 28a, and a small-diameter portion 33c which is rotatably fitted to the small-diameter hole portion 28b. 33d is a positioning reference line (a positioning mark).

As shown in FIGS. 3, 6 and 7, a roughly C-shaped angle scale plate 34 is fixed to the front of the ring-shaped portion 22 of the attaching frame body 21. The angle scale plate 34 prevents the flange 33b from being released from the large-diameter hole portion 28a. Also, an angle scale 34a and an aligning mark 34b are indicated in the angle scale plate 34, and a memory 34c which is used in cooperation with the mark 34b and displays the position of the left and right direction of the lens attaching frame 18 is indicated in the front of the head frame 17.

As shown in FIG. 2, the periphery surfaces of the flange 33b and the small-diameter portion 33c are provided with a cut-circular pattern resistors 35a, 35b continuously provided at one end (refer to FIG. 7). And, the sliding contacts 36, 37 comprised of a plate spring of the conductive material is flexibly contacted to the pattern resistors 35a, 35b. The pattern resistors 35a, 35b and the sliding contacts 36, 37 constitute an axis angle detecting means (information detecting means).

As shown in FIGS. 3 and 6, the lens supporting protrusion 38, 39 are protruded from the front of the rotary lens attaching frame 33 at intervals, and a lens supporting plate spring 40 is fixed to the lens supporting protrusion 38. As shown in FIG. 10(b), the lens supporting plate spring 40 has three plate spring portions 40a, 40b, 40c.

As shown in FIG. 10(b), the lens supporting protrusion 38 has three lens coupling grooves 38a, 38b, 38c, and as shown in FIG. 10(a), the lens supporting protrusion 39 has three lens coupling grooves 39a, 39b, 39c. Also, the lens coupling groove 39a is provided with detecting terminals (detecting means) 41, 42 as the information detecting portion, the lens coupling groove 39b is provided with detecting terminals (detecting means) 43, 44 as the information detecting portion, and the lens coupling groove 39c is provided with detecting terminals (detecting means) 45, 46 as the information detecting portion.

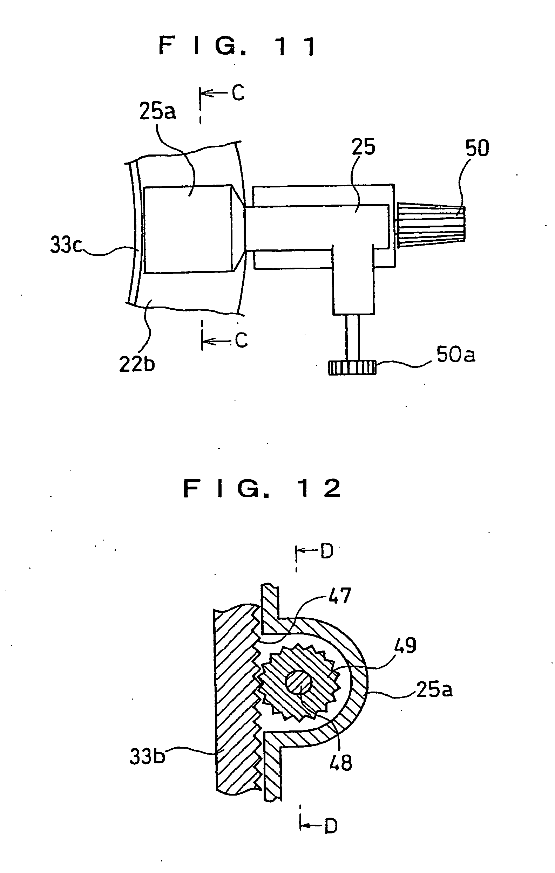

As shown in FIGS. 12 to 14, the rear surface of the flange 33b of the rotary lens attaching frame 33 is provided with a gear 47. A rotary operating shaft 48 is rotatably supported in the bearing portion 25 of the attaching frame body 21, and a pinion 49 fixed to the rotary operating shaft 48 is accommodated in the receiving portion 25a. The pinion 49 is engaged with the gear 47. 50 is an operating handle provided in the outer end of the rotary operating shaft 48, and 50a is an operating shaft fixing screw screwed to the bearing portion 25.

### (Head frame)

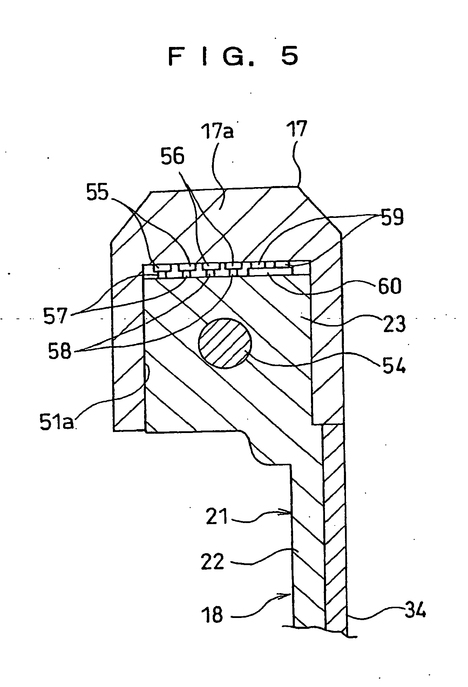

As shown in FIG. 4, the center of the head frame 17 is provided with a nosepiece attaching protrusion 51 which swells upwardly and forwardly. Also, the left and right portions of the head frame 17 are provided with a guide grooves 51a, 51a which is extended leftward and rightward as shown in FIG. 4 and is opened leftward and rightward and in the lower side as shown in FIGS. 4 and 5.

The bearings 52, 53 are inserted and fixed in the both ends of the guide grooves 51a, 51a, and a supporting portion 23 protruded from the upper end of the lens attaching frame 18 is arranged between the bearings 52, 53 as the movable portion. The supporting portion 23 is inserted in the guide groove 51a to be movable leftward and rightward. Also, the both ends of a feed screw 54 penetrating the supporting portion 23 is rotatably supported by the bearings 52, 53. The feed screw 54 is screwed to the penetrating portion of the supporting portion 23, and allows the supporting portion 23 to be moved leftward and rightward by the rotating operation. 55 is an operating handle attached to the outer end of the feed screw 54.

The head frame 17 has the upper walls 17a, 17a located at the both sides of the nosepiece attaching protrusion 51, and the upper wall 17a is provided with a conductive patterns 55, 55 and 56, 56 which are extended lengthwise and face the supporting portion 23, as shown in FIG. 5. Also, the contacts 57, 57 and 58, 58 comprised of the plate spring of the conductive material are elastically contacted with the conductive patterns 55, 55 and 56, 56 which face the supporting portion 23, respectively.

In addition, the upper wall 17a is provided with the pattern resistors 59, 59 which are extended lengthwise and face the supporting portion 23, and a sliding contact 60 contacted with pattern resistors 59, 59 is elastically contacted with the supporting portion 23. The pattern resistors 59, 59 and the sliding contact 60 constitute the left-and-right-moved-position detecting means (information detecting portion) of the supporting portion 23, hence the lens attaching frame 18. The position detecting information from the moved-position detecting means (information detecting portion) can be used in obtaining the inter-pupil distance of the trial frame user. In this case, since the positions of the left and right direction of the left and right lens attaching frames 18, 18 can be individually obtained, the distance from the central position of the left and right direction (center of the left and right direction of the nosepiece) of the head frame 17 to the center of the left and right lens attaching frames 18, 18 can be obtained as the half inter-pupil distance of the trial frame user.

### (Nosepiece supporting structure)

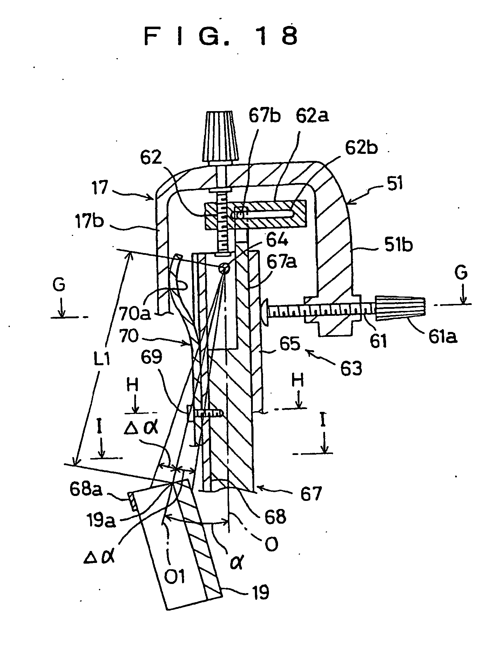

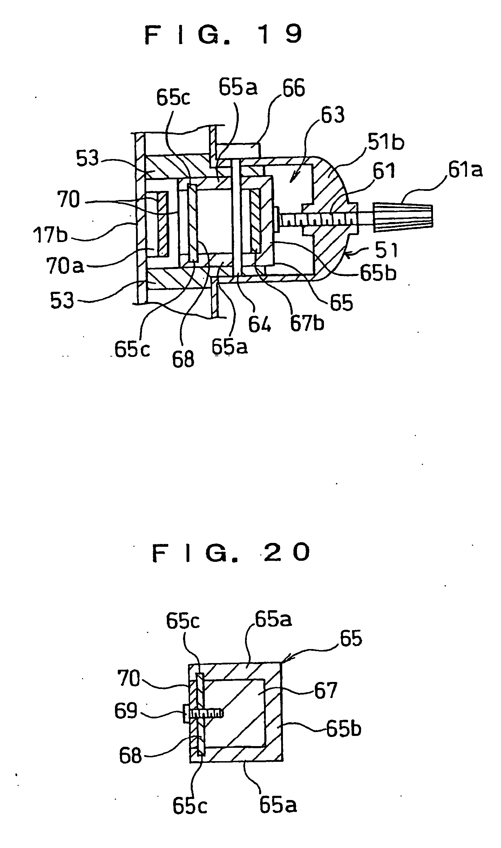

As shown in FIGS. 18 and 19, the nosepiece 19 is attached to the nosepiece attaching protrusion 51 through a nosepiece supporting structure 63. As shown in FIG. 19, the nosepiece attaching structure 63 has a supporting shaft 64 rotatably supported by the bearings 53, 53, and a rotating guide frame (movable portion) 65 which is extended vertically and the upper end thereof is fixed to the supporting shaft 64. Also, a rotated-angle detecting means (information detecting portion) 66 such as potentiometer which detects the rotated amount of the rotating guide frame 65 from the rotation of the supporting shaft 64 is attached to the side of the nosepiece attaching protrusion 51 as a forward and backward position detecting means (nosepiece forward and backward height detecting means) of the nosepiece.

As shown in FIGS. 19 and 20, in the rotating guide frame 65, the cross-section of opposite walls 65a, 65a and an extended wall 65b has the U-shape, and the edge of the opposite walls 65a, 65a is provided with guide grooves 65c, 65c extended upward and downward. Also, the nosepiece elevating member (movable portion) 67 which has a square pillar shape and is extended upward and downward is provided between the opposite walls 65a, 65a of the rotating guide frame 65 to be movable upwardly and downwardly.

A nosepiece forward and backward adjusting screw 61 is screwed to the front wall 51b of the nosepiece attaching protrusion 51. The front end of the nosepiece forward and backward adjusting screw 61 is contacted with the extended wall 65b of the rotating guide frame 65. 61a is an operating handle of the nosepiece forward and backward adjusting screw 61.

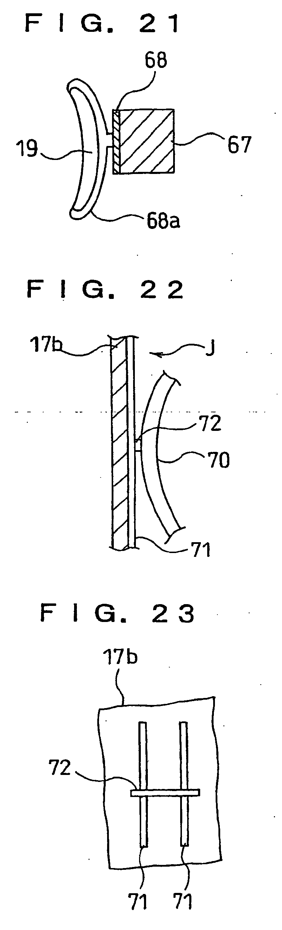

As shown in FIGS. 18 and 20, the nosepiece attaching plate 68 is fixed to the rear surface of the nosepiece elevating member 67 by the screw 69. As shown in FIGS. 18 and 21, the lower end of the nosepiece supporting plate 68 is provided with nosepiece supporting pawl 68a, and the resin nosepiece 19 is supported by the nosepiece supporting plate 68a. In addition, the nosepiece supporting plate 68 is engaged such that the both sides of the guide grooves 65c, 65c are movable upwardly and downwardly.

The plate spring 70 is fixed to the nosepiece supporting plate 68 by the screw 69. The plate spring 70 is curved in the direction distant from the nosepiece supporting plate 68 toward the upper side and then a front portion 70a is curved in the nosepiece supporting plate 68 side. Also, the front portion 70a faces the inner surface of the rear wall 17b of the head frame 17, as shown in FIGS. 18, 19, and 23. As shown in FIGS. 22, 23, the inner surface of the rear wall 17b is provided with a pair of pattern resistors 71, 71 extended upward and downward, and the pattern resistors 71, 71 are contacted with a sliding contact 72 provided in the front end portion 70a of the plate spring 70. The pair of pattern resistors 71, 71 and the sliding contact 72 constitutes the nosepiece vertical position detecting means (information detecting portion).

The nosepiece upper/lower adjusting screw 62 is rotatably supported by the upper wall of the nosepiece attaching protrusion 51. An upper side elevating member 62a is screwed to the nosepiece upper/lower adjusting screw 62, and the upper side elevating member 62a is provided with a slit 62b extended in the forward and backward direction (in the FIG. 18, the left and right direction). Also, the upper portion of the nosepiece elevating member 67 is provided with a thin body portion 67a, and the upper end of the thin portion 67a is attached with a supporting pin 67b inserted to and passing through the slit 62b. In FIG. 18, the supporting pin 67b is inserted passing through the slit 62b such that the supporting pin 67b cannot be moved upward and downward and can be moved in the left and right direction. Accordingly, when the rotating guide frame 65 and the nosepiece elevating member 67 are rotated around the supporting shaft 64, the nosepiece elevating member 67 is driven upward and downward direction with respect to the rotating guide frame 65, and simultaneously, the supporting pin 67b is moved leftward and rightward in the slit 62b.

### (Temple attaching structure)

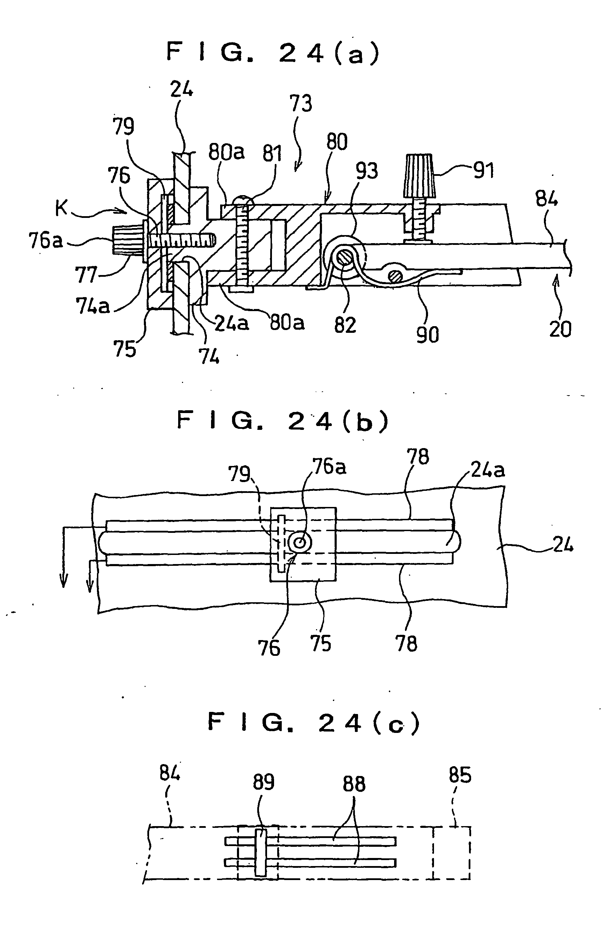

As shown in FIG. 24(a), the temple 20 is attached to the temple attaching portion 20 through the temple attaching structure 73 such that the movement thereof is adjustable leftward and rightward.

The temple attaching structure 73 has a slide supporting member (movable portion) 74 arranged in the rear side of the temple attaching portion 24, a release preventing member (movable portion) 75 arranged in the front side of the temple attaching portion 24, a fixing screw 76 penetrating the release preventing member 75 and screwed to the slide supporting member 74, and a belleville spring 77 intermediated between the handle portion 76a of the fixing screw 76 and the release preventing member 75. The slide supporting member 74 is provided with a protrusion 74a engaged with the guide slit 24a of the temple attaching portion 24 such that the protrusion cannot be moved upwardly and downwardly and can be moved leftward and rightward.

As shown in FIG. 24(b), the front of the temple attaching portion 24 is provided with pattern resistors 78, 78 extended in left and right direction along the vertical direction guide slit 24a, and the release preventing member 75 is provided with a sliding contact 79 contacted with the pattern resistors 78, 78. The pattern resistors 78, 78 and the sliding contact 79 are used as the facial width detecting means (information detecting portion) together with the moved-position detecting means (information detecting portion) comprised of the pattern resistors 59, 59 and the sliding contact 60. Also, the sliding contact 79 is contacted with the pattern resistors 78, 78 by the elastic force of the belleville spring 77.

Also, the temple attaching structure 73 has a temple supporting member 80 provided with plate portions 80a, 80a located at vertical sides of the slide supporting member 74, a vertical supporting shaft 81 penetrating the plate portions 80a, 80a and the slide supporting member 74 and horizontal-rotatably supports the temple supporting member 80 to the slide supporting member 74, a horizontal supporting shaft 82 which is attached to the temple supporting member 80, and a rotated-angle detecting means 83 such as the potentiometer for detecting the rotation of the horizontal supporting shaft 82 as the information detecting portion.

As shown in FIG. 24, the temple 21 has a first arm (movable portion) 84 which is attached to the horizontal supporting shaft 82 so as not to be rotated relatively and moves upwardly and downwardly, a supporting member 85 provided in the free end of the first arm 84 as shown in FIG. 3, a supporting member 86 slidably supported by the first arm 84, and a second arm (movable portion) 87 which one end penetrates the supporting member 85 and is integrally provided in the supporting member 86.

Also, as shown in FIG. 24(c), the first arm 84 is provided with a pair of pattern resistors 88, 88, and the supporting member 86 is provided with a sliding contact 89 contacted with the pattern resistors 88, 88. The pattern resistor 88, 88 and the sliding contact 89 constitute a length detecting means (information detecting portion) of the temple 20.

Also, the first arm 84 of the temple 20 is urged to be rotated upwardly through a torsion coil spring 90 wound and supported by the supporting shaft. Also, an adjusting screw 91 screwed to the temple supporting member 80 is contacted with the upper portion of the first arm 84, thereby the upper-and-lower rotated position of the first arm 84 can be adjusted.

### <Information transmitting circuit>

As shown in FIG. 2, the above-mentioned detecting terminals (detecting means) 31, 32 are connected to a microcomputer chip for data transmission (information outputting means) 92 attached to the lower portion of the rear surface of the attaching frame body (moved-lens attaching frame) 21. Also, the microcomputer chip 92 is connected to the sliding contacts 36, 37 contacted with the cut-circular pattern resistors 35a, 35b, the detecting terminals (detecting means) 41, 42, the detecting terminals (detecting means) 43, 44, and the detecting terminals (detecting means) 45, 46.

In addition, the contacts 57, 57 and 58, 58 slidably contacting with the conductive patterns 55, 55 and 56, 56, and the pattern resistors 59, 59 are connected to the microcomputer ship 92. The rotated-angle detecting means (information detecting portion) 66 such as the potentiometer and the pattern resistors 71, 71 are connected to the conductive patters 55, 55 and 56, 56.

Also, the microcomputer chip 92 is connected to the pattern resistors 78, 78, the rotated-angle detecting means 83 such as the potentiometer, the pattern resistors 88, 88. Also, the lower portion of the attaching frame body 21 is provided with outputting terminals 93, 94 of the microcomputer chip 92.

### <Information receiving circuit>

As mentioned in the above, the frame inserting concave portion sb has a small-diameter concave portion 95 and a large-diameter concave portion 96 which can respectively receive the small-diameter portion 22a and the large-diameter portion 22b in the ring-shaped portion 22. And, a step surface 97 is provided between the small-diameter concave portion 95 and the large-diameter concave portion 96 as a stopper, and the step surface 97 is attached with a micro switch 98. Also, the bottom portion of the large-diameter concave portion 96 is provided with a pair of information inputting terminals 99, 100 as the information inputting portion (information inputting means). The micro switch 98 and the detecting terminals 99, 100 are connected to an operation control circuit 7 which is a computer.

### [Operation]

Hereinafter, the operation of the above-mentioned composition will be described.

### (1) Measurement of the refraction property and preparation of the perfect prescription value

In this system, the refraction property (refraction information) such as the long-sightedness, the short-sightedness or the astigmatism of the tested eye is measured using a subjective eye test apparatus 4 such as a vision tester or an objective autorefractometer 5. The measured refraction property information such as the long-sightedness, the short-sightedness or the astigmatism of the tested eye is inputted to the operation control circuit 7. The operation control circuit 7 allows a memory 10 to store the inputted refraction information, allows an information record reproducing device 12 to record the information, and allows the display means 11 to display the information.

In addition, the operation control circuit 7 prepares the prescription value such as S (cylindrical degree), C (cylindrical degree) and A (axis angle of the cylindrical axis) of the eyeglasses from the inputted refraction property. Also, the obtained prescription value can be referred to as the perfect prescription value because it is prepared to perfectly correct the long-sightedness, the short-sightedness and the astigmatism.

### (2) Determination of the final prescription value of the eyeglass

Next, a wearing test is performed by the lens exchange method using the trial frame 16 based on the prescription value lower than the perfect prescription value, and the final confirmation is performed.

For example, in case where vision 0.2 of an test subject becomes 1.2 by the perfect prescription value of the eyeglasses, the diopter of the perfect prescription value of the eyeglasses having the vision 1.2 becomes -4.5. However, when the vision 0.2 is suddenly corrected to 1.2, the test subject often feels dizziness or headache, or may feel eye fatigue because the correction width is large.

Accordingly, the eyeglass prescription value becomes the value lower than the perfect prescription value obtained by measuring the S, C, A values of the actually prepared eyeglasses, thereby the dizziness, the headache or the eye fatigue is suppressed if at all possible. Here, in case where vision 0.2 of the test subject becomes 1.2 by the perfect prescription value of the eyeglasses, for example, the corrected vision may be 0.8 lower than the vision 1.2 by the perfect prescription value.

### (3) Wearing test based on the eyeglass prescription value

### <Initial set of the exchange lens based on the eyeglass prescription value>

In this case, when prescribing the eyeglasses for obtaining the final vision, for example, the exchange lens (spherical lens) L of -4.5D as the reference lens is contacted with the receiving portion 25a provided in the rear surface of the lens attaching frame 18 and the lens coupling groove 29a, and the exchange lens L is strongly pressed to the receiving portion 25a and the lens coupling groove 29a by the lens supporting plate spring 30. At this time, the reference line (positioning line, that is, the positioning mark) Ld is positioned in conformity with a reference line (positioning line, that is, positioning mark) 22c formed at the ring-shaped portion 22, and the terminals r1, r2 are contacted with the detecting terminals 31, 32 of the lens coupling groove 29a, respectively, thereby, the setting of the reference lens L is finished.

Also, the exchange lens L is engaged with the lens coupling grooves 38a, 39a formed at the lens supporting protrusions 38, 39 of the rotary lens attaching frame 33, and the exchange lens L is pressed by the plate spring portion 40a, thereby the exchange lens L can be supported by the lens supporting protrusions 38, 39. Similarly, the exchange lens L can also be supported by the lens supporting grooves 38b, 39b and the plate spring portion 40b or the lens supporting grooves 38c, 39c and the plate spring portion 40c. At this time, the reference line (positioning line, that is, positioning mark) Ld formed at the exchange lens L fits the reference line (the positioning line, that is, the positioning mark) 33d formed at the rotary attaching frame 33. By this, the terminals r1, r2 of the exchange lens L are contacted with the detecting terminals 41, 42 (43, 44, or 45, 46) of the lens supporting groove 39a (39b or 39c).

Accordingly, in preparation of a plurality of exchange lenses of -0.25D, as mentioned in the above, the exchange lenses L are attached to the rotary lens attaching frame 33 one by one as occasion demands, and the vision is set to be the prescription value of about 0.8 lower than 1.2. In this case, when there is a cylindrical axis, the exchange lens having a cylindrical axis is set.

### <The trial frame wearing and the movable portion adjustment>

At this state, the test subject wears the trial frame 16, the operating handles 33, 33 are turned, the feed screws 23, 23 are rotated such that the left and right lens attaching frames 18, 18 is moved leftward and rightward, and the center of the left and right lens attaching frame 18 is fitted with the center of the left and right eyes of the test subject, and at the same time, the fixing screw 76 is loosened, the slide supporting member 74 is moved left and right such that the interval between the left and right temples 20, 20 becomes the facial width of the test subject, thereby the left and right temples 20, 20 are naturally hung on the ears of the test subject.

Also, when the adjusting screw 91 is rotated to be moved in the lower side against the elastic force of the torsion coil spring 90, the temple 20 is rotated in the lower side, and when the adjusting screw 91 is rotated to be moved in the upper side, the temple 20 is rotated in the upper side by the elastic force of the torsion coil spring 90, thereby the upper and lower angles of the temple 20 are adjusted. And, the first and second arms 84, 87 are frictionally supported each other by the friction resistance generated between the first arm 84 and the supporting member 86 and the friction resistance generated between the second arm 87 and the supporting member 85. Thus, when the second arm 87 is moved forwardly and backwardly with respect to the first arm 84 against the friction resistance, the length of the temple 20 by the first and second arms 84, 87 is flexibly adjusted. By adjusting the angle and length of the temple 20, the temple 20 is naturally hung on the ears of the test subject.

And, by rotating the operating handle 61a to reciprocate the front end of the nosepiece forward and backward adjusting screw 61 with respect to the extended wall 65b of the rotating guide frame 65, the rotating guide frame 65 is rotated in the front and rear. And, by rotating the nosepiece up/down adjusting screw 62 to drive the nosepiece elevating member 67 upward and downward, the position in the forward and backward direction and the position in the upward and downward direction of the nosepiece 19 are adjusted, and the falling down of the lens attaching frames 18, 18, etc. is adjusted.

By adjusting the movable portion, the test subject can naturally wear the trial frame 16 at good state.

### <Correction of the prescription value according to the wearing>

After the trial frame 16 is adjusted, the exchange lens L is exchanged, asking the test subject about the seen state. In case where there is a cylindrical axis, in the state the test subject sees the radial eye test chart using the chart display device 15, the operating handle 50 is rotated, and the rotary lens attaching frame 33 is rotated until the position where the state that the test subject sees the eye test chart becomes constant. As such, the state that the test subject can see well is finally selected.

And, at this state, when the person wears the trial frame 16 attaching a plurality of exchange lenses L for several tens of minutes, if the person does not feel inconvenience, the total diopter of the lens by the reference lens and the exchange lenses at this time becomes the final eyeglass prescription value.

### <The trial frame information and the lens refraction property detection/input> (Information detection)

After the wearing test is finished, the slide members sa, sa provided in the lens receiving drawer 2 of the eye test table 1 are moved leftward and rightward, the interval between the frame inserting concave portions sb, sb provided in the slide members sa, sa is adjusted to be equal to the interval between the left and right lens attaching frames 18, 18 of the trial frame 16, and the lens attaching frames 18, 18 are inserted to the frame inserting concave portions sb, sb.

By this insertion, the micro-switch 98 provided in the step surface 97 is turned ON by the step surface 26 of the lens attaching frame 18. In this position, the output terminals 93, 94 of the microcomputer chip 92 are contacted with the detecting terminals 99, 100.

And, when the signal ON from the micro-switch 98 is inputted to the operation control circuit 7, the operation control circuit 7 judges that the lens attaching frames 18, 18 of the trial frame 16 are inserted to the frame inserting concave portions sb, sb, and allows the microcomputer chip 92 to be conducted through the detecting terminals 99, 100 and the output terminals 93, 94.

By this conduction, the microcomputer chip 92 is operated and sequentially detects the movable portion information of the trial frame 16 and the information such as the refraction property of the exchange lens.

As the detected information of the movable portion of the trial frame 16, there are a resistor values of the pattern resistors 59, 59 conducted by the sliding contact 60, the rotated angle signal from the rotated-angle detecting means 66, the resistor values of the pattern resistors 71, 71 the friction resistance by the sliding contact 72, the resistor values of the pattern resistors 78, 78 the friction resistance by the sliding contact 79, the rotated angle signal from the rotated-angle detecting means 83, and the resistor values of the pattern resistors 88, 88 the friction resistance by the sliding contact 89.

As the information such as the refraction property of the exchange lens, there are the resistor values of the pattern resistor 35a, 35b between the sliding contacts 36, 37, the resistor value of the resistor R of the exchange lens L contacted with the detecting terminals 41, 42, the resistor value of the resistor R of the exchange lens contacted with the detecting terminals 43, 44, and the resistor value of the resistor R of the exchange lens contacted with the detecting terminals 45, 46.

### (Detected information transmission)

And, when the microcomputer chip 92 sequentially detects the information of the movable portion of the trial frame 16 and the information such as the refraction property of the exchange lens, the detected signal is outputted from the output terminals 93, 94, and the outputted detected signal is inputted to the operation control circuit 7 through the detecting terminals 99, 100.

In addition, the detected signal may be inputted directly to the operation control circuit 7 by wire, without using the detecting terminals 99, 100. In this case, since the microcomputer chip 92 does not have to be provided in the trial frame 16, the weight of the trial frame 16 can be somewhat reduced.

Also, as mentioned in the above, in the wearing test of the trial frame 16, when the test subject see the radial eye test chart using the chart display device 15, the display switchover of the eye test chart of the chart display device 15 is generally performed using a remote controller. The signal for display switchover of the eye test chart outputted from the remote controller may be transmitted to the chart display device 15 by a wireless communication means in providing a wireless communication means such as infrared rays or electric waves to the remote controller. In this case, the chart display device 15 is provided with the wireless receiving means which receives the wireless such as infrared rays or electric waves, and receives the signal from the remote controller. In this wireless communication, the Bluetooth technique can be used.

Also, the trial frame 16 may be mounted with a power supply such as a battery (not shown) and at the same time, may be provided with a wireless communication means which transmits or receives the wireless such as infrared rays or electric waves. And, the information detected by the microcomputer chip 92 may be transmitted from the trial frame 16 by the wireless communication means, and may be received by the wireless receiving means of the chart display device 15. And, the received information may be transmitted from the chart display device 15 to the operation control circuit 7.

Also, the information detected by the microcomputer chip 92 may be transmitted to the remote controller by the wireless communication means, and may be transmitted from the remote controller to the chart display device 15 by the wireless communication means. In this wireless communication, the Bluetooth technique can be used. In this case, since the detected information can be transmitted in real time, the test subject or a testing person performs the definite operation of the detected information when the fitting by the trial frame 16 is decided. Also, the trial frame 16 and the remote controller may be connected by wire using an attachable/detachable connector.

In this case, since the battery or the microcomputer chip 92 may be provided in the remote controller side, the weight of the trial frame 16 can be reduced, and simultaneously, since the remote controller can be separated from the trial frame 16 when desiring to use only the chart display device 15, the trial frame 16 is not disturbed.

Also, a memory card for storing the detected information can be attached or detached to the trial frame 16, and the detected information is stored in the memory card, thereby the contents stored in the memory card may be taken out by the personal computer. In this case, the detected information can be easily taken out.

Also, in the wearing test using the trial frame 16, as mentioned in the above, the exchange lens, that is, the exchange lens of the perfect correction value is mounted in the trial frame 16 as a main inspected lens, and a plurality of exchange lenses having the low degree are combined with respect to the main exchange lens, thereby the prescription value (eyeglass prescription value) of the eyeglass lens having the degree lower than that of the eyeglass lens of the perfect correction value (perfect prescription value) may be obtained.

Accordingly, by the display as explained later, the exchange selection of the combination of the exchange lens is facilitated.

In other words, for this, each refraction properties (S, C, A) of the plurality of exchange lens now set to the trial frame 16 may be displayed at the chart display device 15. This information is detected by the above-mentioned method, and is obtained by the later-mentioned method.

By this display, in case of desiring to change the combination of the exchange lens in the test using the trial frame 16, since the kind and degree of the exchange lens now set to the trial frame 16 can be known by seeing the chart display device 15, the combination of the exchange lens can be easily changed. Also, in addition to such display, the total refraction properties S, C, A of the plurality of exchange lenses now set to the trial frame 16 may be displayed at the chart display device 15. This information is detected by the above-mentioned method, and is obtained by the later-mentioned method.

Also, when such display is performed, the perfect correction value of the eyeglass lens or the corrected vision value of the tested eye of the test subject of the test subject by the perfect correcting value, and the corrected vision value of the tested eye by the prescription value (eyeglass prescription value) of the eyeglass lens obtained by the trial frame 16 are displayed at the chart display device 15, thereby they can be used as the reference for obtaining the final corrected vision.

Also, such display may be displayed at the chart display device 15 together with the eye test chart or independently.

Also, the information (later-mentioned frame information) of the movable portion of the trial frame 16 is displayed, and thus, is used as data (information) for selecting the eyeglass frame.

Also, the operation control circuit 7 is connected to the wireless receiving means, and the information detected by the microcomputer chip 92 may be directly transmitted from the trial frame 16 to the operation control circuit 7 by the wireless communication means.

### (Detected information operation/display)

When the detected signal is inputted from the microcomputer chip 92, the operation control circuit 7 obtains the information (frame information) of the movable portion of the trial frame 16 shown in the following (a) and (b) and the information (exchange lens information) such as the refraction property of the exchange lens by the operation from the inputted detected signal,

### (a) Frame information

The position information of the left and right direction of the lens attaching frames 18, 18 is obtained from the resistor values of the pattern resistors 59, 59 conducted by the sliding contact 60. Since the position information of this case can be individually obtained every left and right lens attaching frames 18, 18, the distance from the central position of the left and right direction of the head frame 17 (center of the left and right direction of the nosepiece) to the center of the left and right lens attaching frames 18, 18 can be obtained as the half inter-pupil distance of the trial frame user. Also, since the position information of the left and right direction of the left and right lens attaching frames 18, 18 can be obtained individually, it can be exactly obtained even though the trial frame user has a severe heterophoria.

Here, the distance from the supporting shaft 64 to the contact portion 19a of the front end nosepiece 19 is Lx, the perpendicular axis when the rotating guide frame 65 is at perpendicular state is O, the line passing through the supporting shaft 64 and the front end contact portion 19a is O1 and the angle formed by the lines O, O1 is α. The distance Lx and the angle *α* are the known values. Thus, if the position of the forward and backward direction of the nosepiece 19 when the rotating guide frame 65 is at perpendicular state is the reference position, the rotated angle of Δ α in the forward and backward direction of the rotating guide frame 65 by the nosepiece forward and backward adjusting screw 61 is obtained from the rotated angle signal of the rotated-angle detecting means 66. And, the position of the forward and backward direction of the nosepiece 19 is obtained from the rotated angle Δ α , the distance Lx, and the angle α .

The position of the vertical direction of the nosepiece 19 is obtained from the resistor values of the pattern resistors 71, 71 conducted by the sliding contact 72. The position of the left and right direction of the slide supporting member 74, that is, the position of the base of the left and right temples 20, 20 is obtained from the resistor values of the pattern resistors 78, 78 conducted by the sliding contact 79. The tilt angle of the temple is obtained from the rotated angle signal from the rotated-angle detecting means 83. The length of the temple 20 is obtained from the resistor values of the pattern resistors 88, 88 conducted by the sliding contact 89.

And, the operation control circuit 7 obtains the width between the temples 20, 20 (so-called, the width corresponding to the facial width) from the position information of the left and right direction of the lens attaching frames 18, 18 and the position information of the left and right direction of the slide supporting member 74.

Also, the operation control circuit 7 allows the width between the temples 20, 20 obtained by the above-mentioned method, the position or the height of the forward and backward direction of the nosepiece, and the length of the temple 20 to be displayed at the display means 11 as the eyeglass frame selecting information (fitting information when wearing the eyeglasses), as shown in FIG. 26.

Accordingly, the eyeglass frame selecting information can be obtained when performing the wearing test by the trial frame 16 for determining the final lens prescription value.

As the result, since the customer does not have to wear the eyeglass frame actually when selecting the frame, it is sufficient that the eyeglass frame displayed on the monitor by the catalog or personal computer is selected. Also, since the eyeglass frame information can be used when selecting a new eyeglass frame, the eyeglass frame information can be reused by stocking the information once in the opticians as the data. Thereby, since the customer can contact the opticians using the Internet and select and order the eyeglass frame on the monitor by stocking the eyeglass frame information once in opticians as the data, the customer does not have to go to opticians when the customer wants to make a new eyeglass frame.

### (b) Exchange lens information

In case where the resistor value of the resistor R of the exchange lens L contacted with the detecting terminals 41, 42, the resistor value of the resistor R of the exchange lens L contacted with the detecting terminals 43, 44, and the resistor value of the resistor R of the exchange lens L contacted with the detecting terminals 45, 46 are not less than the predetermined value, the exchange lens L is judged to be the cylindrical lens, and the cylindrical degree of the exchange lens L is obtained. Also, in case where the resistor value of the resistor R of the exchange lens L contacted with the detecting terminals 41, 42, the resistor value of the resistor R of the exchange lens L contacted with the detecting terminals 43, 44, and the resistor value of the resistor R of the exchange lens L contacted with the detecting terminals 45, 46 are less than the predetermined value, the exchange lens L is judged to be the spherical lens, the spherical degree of the exchange lens L is obtained.

In the case that the exchange lens L is judged to be the cylindrical lens, the rotated angle of the rotary attaching frame 18, that is, the angle of the cylindrical axis of the exchange lens L is obtained from the resistor values of the pattern resistors 35a, 35b between the sliding contacts 36, 37.

And, the operation control circuit 7 allows the refraction degree or the angle of the cylindrical axis and the cylindrical degree of the plurality of exchange lenses obtained as mentioned in the above to be displayed at the display means 11 as shown in FIG. 26, and uses them as the final prescription value of the actual eyeglasses.

Also, in the present example, as mentioned in the above, the trial frame is provided with the information detecting portion, and an input means for inputting the information from the information detecting portion to the computer is provided. Also, as the information detecting portion, similar to the present example, the composition which the information of the movable portion of the trial frame 16 for the fitting is detected and the composition that the information detecting portion for detecting the refraction property of the exchange lens is provided is preferable. However, the present invention is not limited to the composition, but it may be selected any one of the composition which the information of the movable portion of the trial frame 16 for the fitting is detected and the composition that the information detecting portion for detecting the refraction property of the exchange lens is provided.

### (Modified example 1)

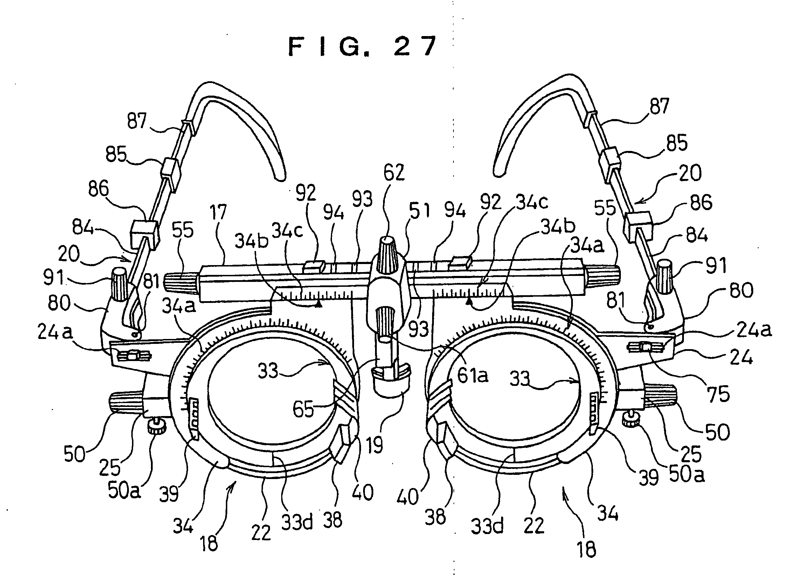

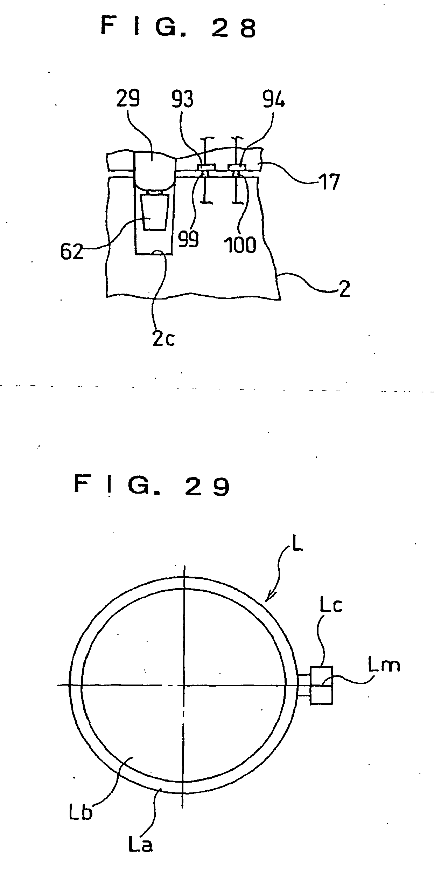

Also, the microcomputer chip 92 or the output terminals 93, 94 may be provided in the head frame 17, as shown in FIG. 27. In this case, one point of the lens receiving drawer 2 is provided with the head frame receiving portion 2c such that the frame information or the exchange lens information equal to the above-mentioned information can be detected when the head frame 17 is inserted and arranged (set) as shown in FIG. 28. In this case, since the slide members sa, sa do not have to be provided as mentioned in the above and the information can be detected regardless of the interval between the lens attaching frames 18, 189, the structure for detection becomes simple.

### (Modified example 2)

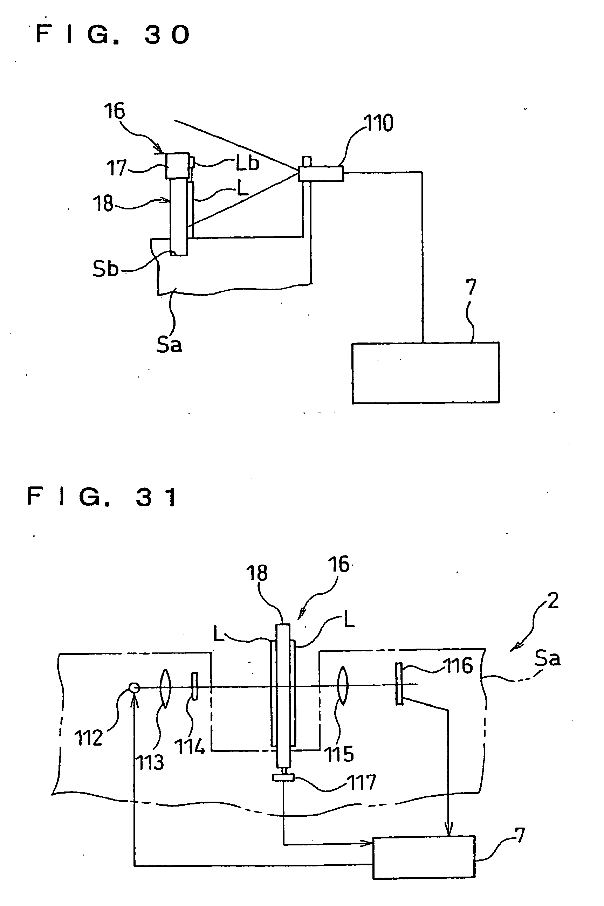

Also, as shown in FIG. 29, the handle Lc of the exchange lens L is provided with a mark Lm such that the mark Lm provided in the handle La of the exchange lens L of the lens frame 18 may be photographed by a CCD camera 110 in FIG. 30 to obtain the direction of the cylindrical axis of the exchange lens L from the photographed picture by the operation control circuit 7 when the lens attaching frame 18 of the trial frame 16 is inserted and arranged (set) to the frame receiving portion sb of the lens receiving drawer 2. Also, for convenience, the trial frame 16 and the lens attaching frame 18 are schematically shown.

### (Modified example 3)

Also, it may be provided the composition that when the lens attaching frame 18 of the trial frame 16 is inserted and arranged (set) to the frame receiving portion sb of the lens receiving drawer 2, a measurement optical system for measuring the spherical degree of the plurality of exchange lenses of the lens frame 18 or the refraction properties such as the direction of the cylindrical axis or the cylindrical degree is provided in the lens receiving drawer 2 as shown in FIG. 31. The measurement optical system has a measurement light-projecting optical system and a light-receiving optical system.

The measurement optical system has a light source 112, a collimator lens 113 for changing the measured beam of the light source 112 to a parallel beam, and a pattern plate 114 for changing the parallel beam to a pattern beam. In the pattern plate 114, a ring-shaped pattern or a triangular pattern, etc. can be used, and a lens array plate in which a plurality of small lenses are provided in parallel can be used. Also, the light-receiving optical system has an image forming lens 115 and an area sensor (two-dimensional CCD) 116. Since the above-mentioned measurement optical system can adopt the equivalent to the optical system of the lens meter in principle, the detailed explanations thereof are omitted. Also, for convenience, the trial frame 16 and the lens attaching frame 18 are schematically shown.

In addition, a switch 117 is provided in the bottom portion of the frame receiving portion sb of the lens receiving drawer 2, and the switch 117 is pressed by the lens attaching frame 18 when the lens attaching frame 18 of the trial frame 16 is inserted and arranged (set) to the frame receiving portion sb. And, the ON/OFF signal of the switch 117 is inputted to the operation control circuit 7, and the light source 112 is lighted when the switch 17 is pressed, and then the operation control circuit 7 obtains the refraction property of the plurality of exchange lenses L mounted in the lens attaching frame 18 from the measured (detected) signal from the area sensor 116. In this case, since the means for reading the information of the exchange lens L mounted in the lens attaching frame 18 does not have to be provided in the lens attaching frame 18 or the exchange lens L, the composition of the trial frame 16 becomes simple.

### (Modified example 4)

In the above-mentioned example, in FIG. 18, the operating handle 61a of the nosepiece forward and backward adjusting screw 61 is rotated, the nosepiece forward and backward adjusting screw 61 advances and retreats in the axis direction, and thus, the pressing power of the nosepiece forward and backward adjusting screw 61 or the elastic force of the plate spring 70 allows the rotating guide frame 65 and the nosepiece elevating member 67 to rotate around the supporting shaft 64, but the present invention is not limited to that.

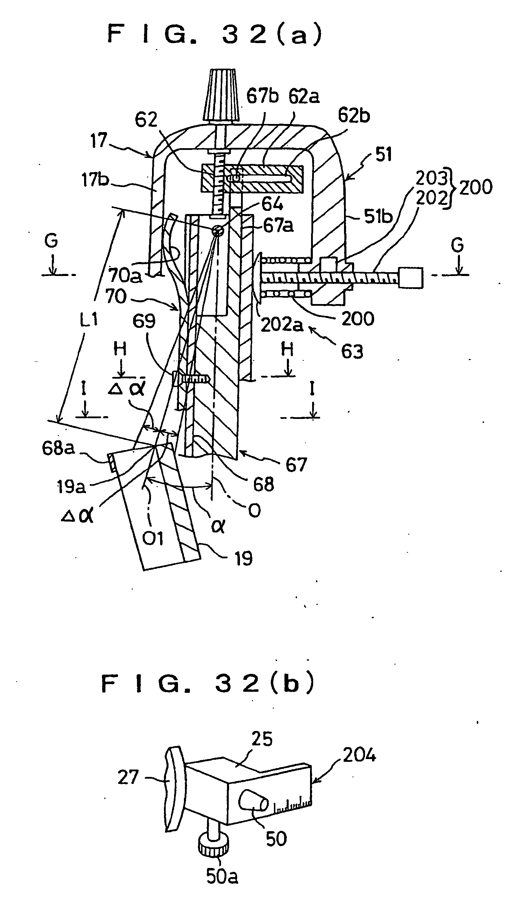

For example, as shown in FIG. 32(a), a coil spring 200 which urges the rotating guide frame 50 and the nosepiece elevating member 67 to rotate around the supporting shaft 64 clockwise in FIG. 32(a) may be provided, and a magnescale 201 for detecting the rotated amount may be intermediated between the front wall 51b of the nosepiece attaching protrusion 51 and the rotating guide frame 50. The magnescale 201 has a magnetic scale 202 penetrating the front wall 51b in the forward and backward direction and a magnetic head 203 attached to the front wall 51b. The end of the rotating guide frame 65 of the magnetic scale 202 is provided with a flange 202a of which the end has the spherical shape, and the coil spring 200 is intermediated between the flange 202a and the end wall 51b. Thus, the spherical-shaped end surface of the flange 202a of the magnetic scale 202 is strongly pressed by the elastic force of the coil spring 200 with respect to the rotating guide frame 65, and the rotating guide frame 50 and the nosepiece elevating member 67 are urged rotatably around the supporting shaft 64 clockwise in FIG. 32(a). Also, it is not shown in the drawing, but the magnetic head 203 is connected to the above-mentioned wire 56, and the output from the magnetic head 203 is inputted to the operation control circuit 7 using the wire 56.

Also, in this composition, the rotating guide frame 65 is rotated clockwise by the elastic force of the coil spring 200, and thus, the position when the handle 202b of the outer end of the magnetic scale 202 is contacted with the front wall 51b becomes the reference position. Also, the moved amount when the magnetic scale 202 is moved from the reference position to the axis (the right direction in FIG. 32(a)), the relationship data of the rotated angle α around the supporting shaft 64 of the rotating guide frame 65, and the position data from lens attaching frame 18 of the nosepiece 19 corresponding to the rotated angle α are previously stored to the memory 10 of the control circuit 6.

Thereby, when a user of the trial frame 16 lays the nosepiece 19 on his/her nose and presses the front wall 51 to the his/her facial side by his/her hand, the rotating guide frame 65 and the nosepiece elevating member 67 is rotated around the supporting shaft 64 counterclockwise in FIG. 32(a), and the magnetic scale 202 slides in the direction protruding in the right side in FIG. 32(a) with respect to the front wall 51b. At this time, the moved amount with respect to the front wall 51b of the magnetic scale 200 is read by the magnetic head 203 to be inputted to the operation control circuit 7. And, the operation control circuit 7 obtains the moved amount with respect to the front wall 51b of the magnetic scale 202 from the signal detected from the magnetic head 203, and obtains the position of the forward and backward direction of the nosepiece 19 with respect to the lens attaching frame 18 from the obtained moved amount.

At this time, divisions 204 for showing the distance from the lens attaching frame 18 to the rear side is provided in the side of the bearing portion 25, and the front wall 51 is moved forwardly and backwardly with respect to the his/her face such that the distance from the top of the cornea of the tested eye of the person wearing the trial frame 16 to the lens attaching frame 18 becomes about the predetermined distance (for example, about 12 mm). This is because the prescription value such as the refraction degree of the refraction value of the eyeglass lens is set such that the tested eye becomes the predetermined vision value, at the state that the distance from the rear side refraction surface of the eyeglass lens of the general eyeglasses to the cornea of the tested eye becomes the predetermined distance (generally, about 12 mm).

### (Modified example 5)

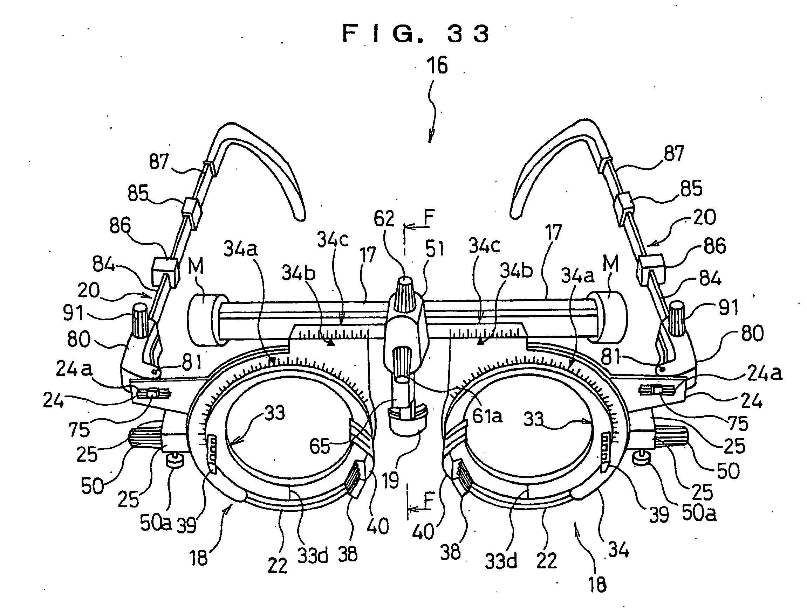

Also, as shown in FIG. 33, it may be provided the composition that the driving motor M is fixed to the head frame 17 and the feed screw 54 in FIG. 4 is rotated by the driving motor M, instead of fixing the operating handle 55 shown in FIG. 3 to the driving motor M. In this case, when the inter-pupil distance is previously known by the test subject's tested eye, the inter-pupil distance is inputted to the operation control circuit 7, and the operation control circuit 7 drives and controls the driving motor M such that the distance between the centers of the lens attaching frames 18, 18 shown in FIGS. 3 and 4 becomes the inter-pupil distance of the test subject. Thereby, the interval of the lens attaching frames 18, 18 can be simply and quickly adjusted.

### (Modified example 6)

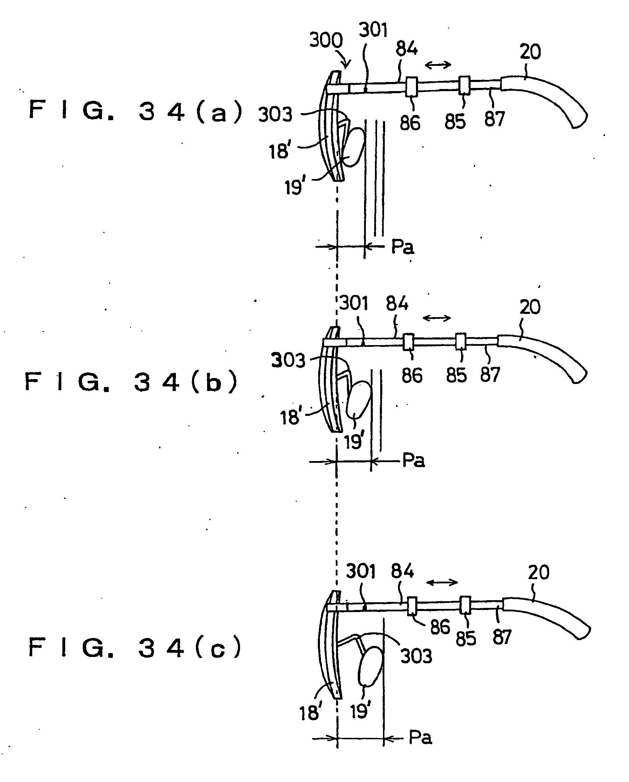

Also, apart from the above-mentioned trial frame 16, a plurality of simple trial frames (eyeglass frame for test) 300 as shown in FIG. 34 may be provided. The trial frames 300 are not provided in order to correct the degree of the eyeglass lens, but to obtain the protruded amount of the nosepiece 19' protruded from the lens attaching frame 18' and the length of the temple 20. Thus, when the test subject wears the trial frames 300 in which the protruded amount Pa of the nosepiece 19' protruded from the lens attaching frame 18' is sequentially increased as shown in FIGS. 34(a)-(c), the trial frames 300 in which the distance from the lens attaching frame 18 to the test subject's cornea becomes the predetermined distance Δx (for example, about 12 mm) is selected in FIGS. 34(a)-(c), and the length of the temple 20 becomes most fitted with the test subject by the method equal to the above-mentioned example.

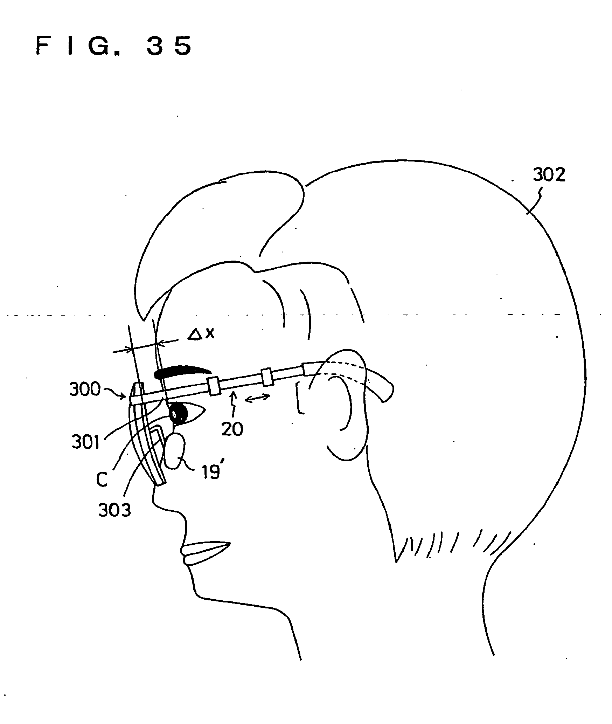

At this time, as shown in FIG. 35, the scale line 301 located at the position where the distance from the lens attaching frame 18' becomes about 12 mm is provided in the side of the temple 20, and it may be selected that the top of the cornea of the tested eye C of the test subject 302 becomes equal to the scale line 301.

Also, in the present modified example, only the temple 20 employs the structure of the above-mentioned example. And, as the length detecting means (information detecting portion) for detecting the length of the temple 20, the pattern resistors 88, 88 and the sliding contact 89 of the above-mentioned example are used. Also, the contact point for taking the signal from the length detecting means (information detecting portion) comprised of the pattern resistors 88, 88 and the sliding contact 89 is provided in the lens attaching frame 18' as similar as the above-mentioned example, the signal detected from the length detecting means (information detecting portion) of the temple 20 is taken from the lens attaching frame 18' using the contact point, and the taken signal is inputted to the operation control circuit 7 as mentioned in the above.

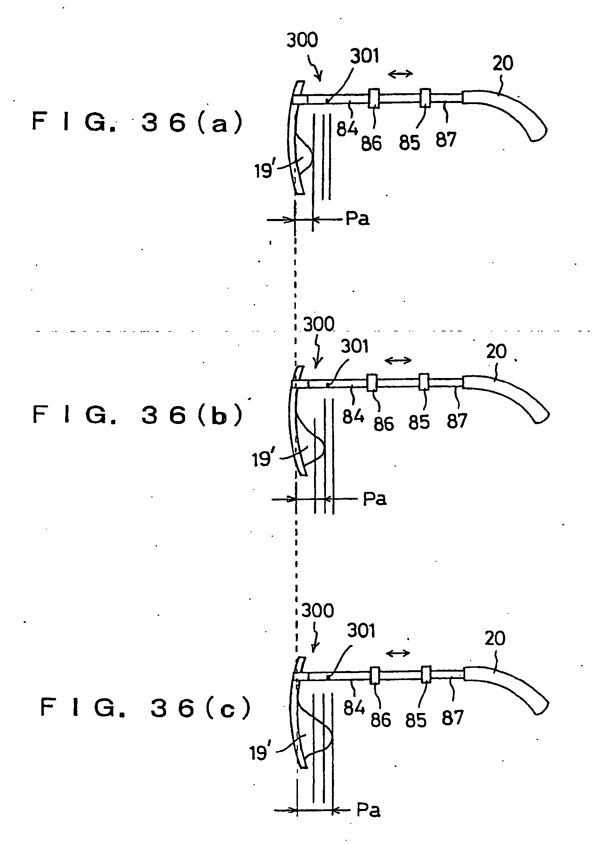

Also, in FIGS. 34 and 35, the nosepiece 19' is mounted in the lens attaching frame 8 through a line-shaped member 303, but the present invention is not limited to that. For example, as shown in FIG. 36, the nosepiece 19' in FIGS. 34 and 35 may be provided directly in the lens attaching frame 18'. Also, in the present modified example, the lens attaching frame 18 is not necessarily needed. For example, it may be the composition that the head frame 17 shown in FIG. 3 and the nosepiece 19' as the same as the present example located at the center of the left and right direction of the head frame 17 are provided and at the same time, the both ends of the head frame 17 is provided with the flexible temple 20 as shown in FIG. 3.

As mentioned in the above, the trial frame 16 or 300 which is an eyeglass frame for test comprises the movable portion for changing and adjusting the size and contour such as the length of the temple 20, the protruded amount of the nosepieces 19, 19' and the interval of the temples 20, 20 according to the user's facial size and contour. Also, since the trial frame 16 or 300 has the composition that detects the data from movable portion on said eyeglass frame for test by the information detecting portion, though the customer does not wear a plurality of eyeglass frames when selecting the eyeglass frame, the fitting eyeglass frame can be finally obtained. As the result, it is sufficient that the eyeglass frame displayed on the monitor by the personal computer or the catalog is selected. Also, since the eyeglass frame information can be stored as record or memory, it can be used when selecting the next eyeglass frame, and thus, it can be reused by stocking the eyeglass frame information once in the opticians as the data. Therefore, since the customer can connect the opticians using the Internet and select and order the eyeglass frame on the monitor, the customer does not have to go to opticians when the customer wants to make a new eyeglass frame. The above-mentioned effect is the same as the following modified example.

### (Modified example 7)

### [Composition]

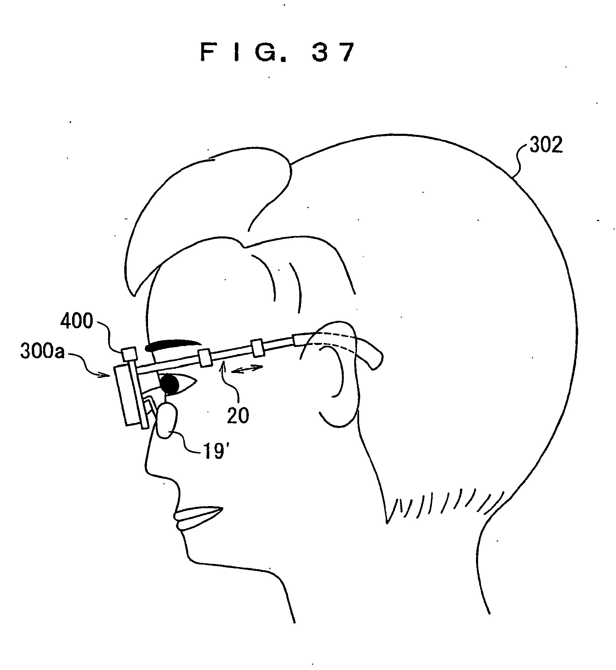

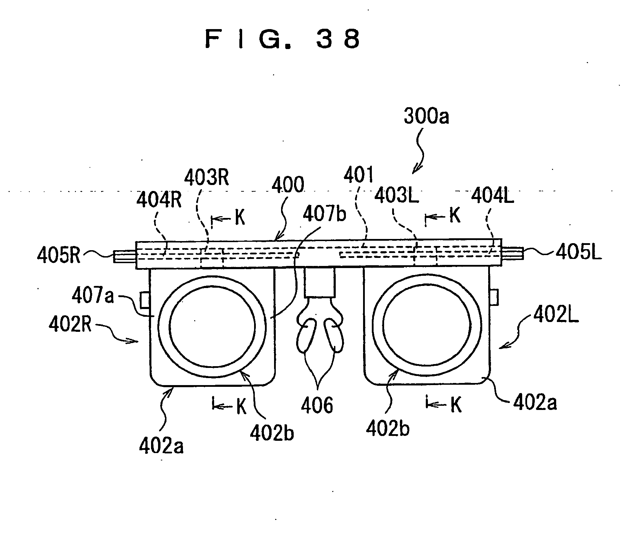

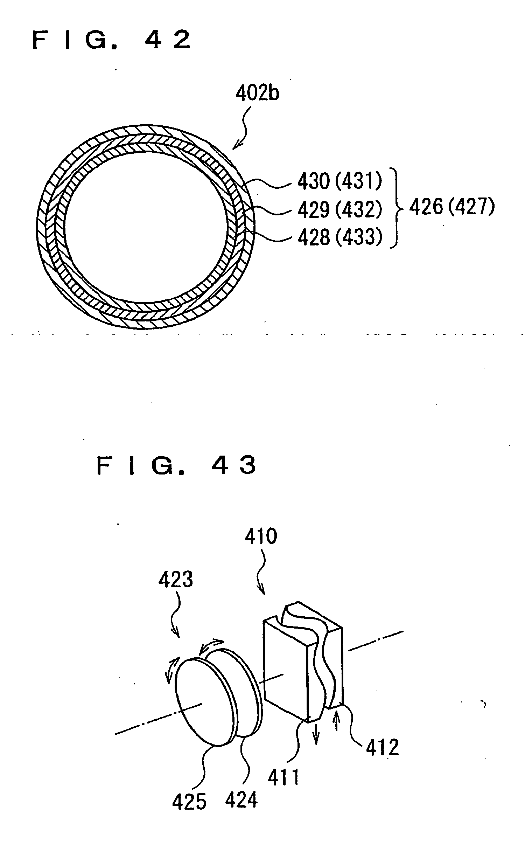

FIGS. 37-43 show the eyeglass frame for test 300a of the modified example 7 according to the present invention. In the present example, the eyeglass frame for test 300 shown in FIG. 37 has a head frame 400 extended leftward and rightward as shown in FIG. 38. The head frame 400 is provided with a guide groove 401 extended leftward and rightward and opened in the lower side, and protrusions 403R, 403L of lens attaching frames 402R, 402L are provided in the left and right sides of the guide groove 401 so as to be movable leftward and rightward. The lens attaching frames 402R, 402L are attached with the above-mentioned temples 20, 20. Also, the same portions as the above-mentioned composition are attached with the same reference numerals and the descriptions thereof are omitted.

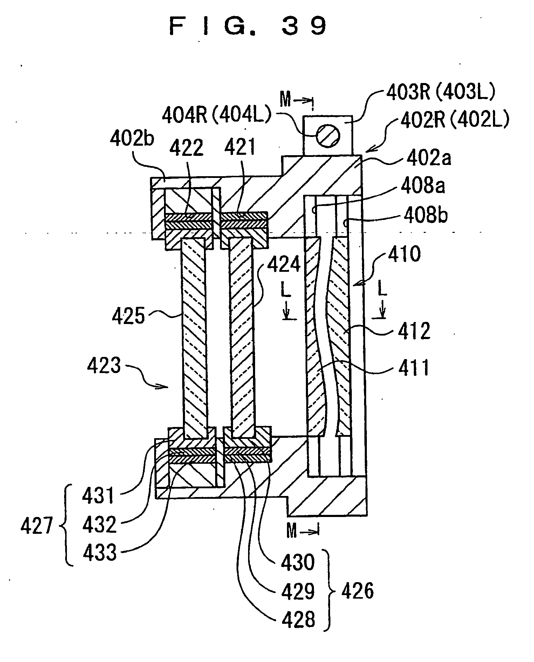

Also, feed screws 404R, 404L extended leftward and rightward are rotatably supported by the left and right portions of the head frame 400. The feed screws 404R, 404L are screwed to the protrusions 403R, 403L and are adjustably moved leftward and rightward by rotating the operation handles 405R, 405L integral with the feed screws 404R, 404L. Also, the central portion of the left and right direction of the head frame 400 is attached with a nosepiece 406. Since the compositions of the optical systems in the lens attaching frame 402R, 402L are equal each other, only the lens attaching frames 402R is described, and the composition of the lens attaching frame 402L is attached with the same reference numerals as that of the lens attaching frame 402R, and the descriptions thereof are omitted. Also, as shown in FIG. 39, the lens attaching frame 420R is provided with a first lens attaching portion 402a and a second barrel-shaped lens attaching portion 402b.

### <First lens attaching portion 402a>

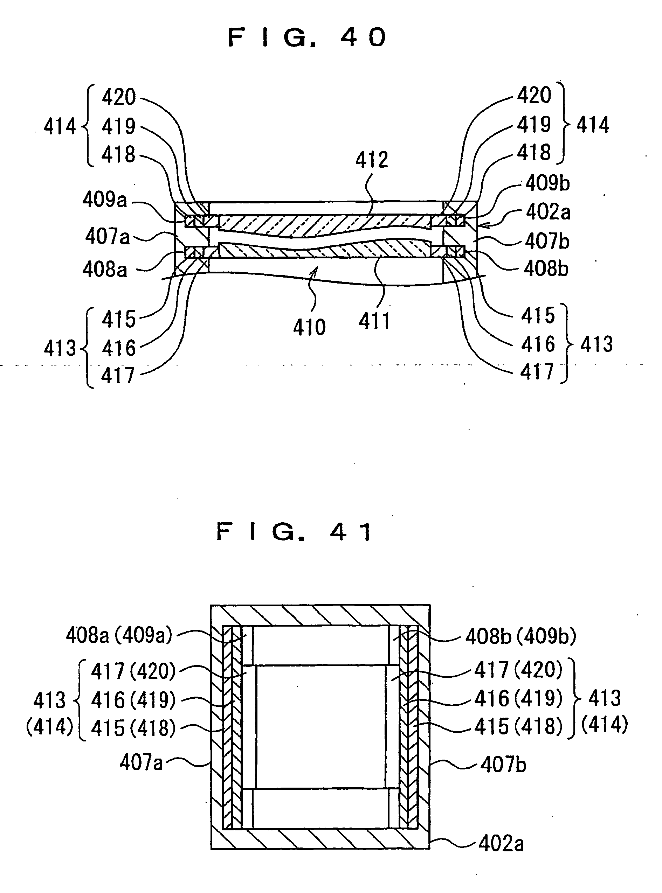

The first lens attaching portion 402a has side wall portions 407a, 407b at the left and right, the side wall portion 407a is provided with guide grooves 408a, 409a which are extended in the vertical direction as shown in FIGS. 40 and 41, and the side wall portion 407b is provided with guide grooves 408b, 409b extended in the vertical direction corresponding to the guide grooves 408a, 409a.

A variable focus lens 410 referred to as Alvarez lens is provided between the side wall portions 407a, 407b. The variable focus lens 410 has a pair of aspherical lenses 411, 412, and the focus thereof can be changed by relatively moving the aspherical lenses 411, 412 in the vertical direction.

Also, the ultrasonic linear motors (ultrasonic motors) 413, 414 are provided in the guide grooves 408a, 408b, respectively.

The ultrasonic linear motors 413, 414 have a piezoelectric element array (oscillation generating member) 415 formed in the straight shape by alternatively connecting a plurality of electrodes (not shown) with piezoelectric elements, a straight-shaped oscillating body (stator) 416 which is provided with a plurality of teeth (not shown) arranged lengthwise in the opposite side to the piezoelectric element array 415 and is oscillated by the piezoelectric element array 415, and a mover 417 frictionally engaged with the plurality of teeth of the oscillating body 416. And, the piezoelectric element array 415 is attached to the oscillating body 416, and the movers 417, 417 of the guide grooves 408a, 408b are fixed to the both sides of the aspherical lens 411.

In this composition, the oscillated phase of the inflection standing wave (traveling wave) generated in the teeth (not shown) side of the stator 416 can be changed by controlling the voltage applied to each electrodes of the piezoelectric element array 415. By changing the phase, the teeth (not shown) of the stator 416 drives the mover 417 in the upper side or in the lower side. In the structure of the ultrasonic linear motor 413, the structure of the known ultrasonic motor can be employed.

Similarly, the ultrasonic linear motor 414 has a piezoelectric element array (oscillation generating member) 418 formed in the straight shape by alternatively connecting a plurality of electrodes (not shown) with piezoelectric elements, a straight-shaped oscillating body (stator) 419 which is provided with a plurality of teeth (not shown) arranged lengthwise in the opposite side to the piezoelectric element array 418 and is oscillated by the piezoelectric element array 418, and a mover 420 frictionally engaged with the plurality of teeth of the oscillating body 419. And, the piezoelectric element array 418 is attached to the oscillating body 419, and the movers 420, 420 of the guide grooves 409a, 409b are fixed to the both sides of the aspherical lens 412.

In this composition, the oscillated phase of the inflection standing wave (traveling wave) generated in the teeth (not shown) side of the stator 419 can be changed by controlling the voltage applied to each electrodes of the piezoelectric element array 418. By changing the phase, the teeth (not shown) of the stator 419 drives the mover 420 in the upper side or in the lower side. In the structure of the ultrasonic linear motor 414, the structure of the known ultrasonic motor can be employed.

### <Second lens attaching portion 402b>

The second lens attaching portion 402b is formed in the cylinder shape, and a pair of ring-shaped grooves 421, 422 are provided in the inner periphery surface of the second attaching portion 402b at intervals in the axis direction. Also, a Vcc lens (variable cross cylinder lens) 423 is provided in the second lens attaching portion 402b.

The Vcc lens 423 has a pair of cylinder lenses 424, 425, and the cylinder lenses 424, 425 are rotated by the ultrasonic motors 426, 427 arranged in the ring-shaped grooves 421, 422.

The ultrasonic motor 426 has a piezoelectric element array (oscillation generating member) 428 formed in the ring shape by alternatively connecting a plurality of electrodes (not shown) with piezoelectric elements, an ring-shaped oscillating body (stator) 429 which is provided with a plurality of teeth (not shown) arranged in the circumferential direction in the opposite side to the piezoelectric element array 428 and is oscillated by the piezoelectric element array 428, an ring-shaped mover 430 frictionally engaged with the plurality of teeth of the oscillating body 429. And, the piezoelectric element array 428 is attached to the outer periphery surface of the oscillating body 429, and the cylinder lens 424 is fixed to the mover 430 of the ring-shaped groove 421.

In this composition, the phase of the traveling wave generated in the teeth (not shown) side of the stator 429 can be changed by way of controlling the voltage applied to each electrodes of the piezoelectric element array 428. By changing the phase, the teeth (not shown) of the stator 429 rotate the mover 430 forwardly or reversely. In the structure of the ultrasonic linear motor 426, the structure of the known ultrasonic motor can be employed.

Similarly, the ultrasonic motor 427 has a piezoelectric element array (oscillation generating member) 431 formed in the ring shape by alternatively connecting a plurality of electrodes (not shown) with piezoelectric elements 431, an ring-shaped oscillating body (stator) 432 which is provided with a plurality of teeth (not shown) arranged in the circumferential direction in the opposite side to the piezoelectric element array 431 and is oscillated by the piezoelectric element array 431, and a ring-shaped mover 433 frictionally engaged with the plurality of teeth of the oscillating body 432. And, the piezoelectric element array 431 is attached to the outer periphery surface of the oscillating body 432, and the cylinder lens 425 is fixed in the mover 433 of the ring-shaped groove 422.

In this composition, the phase of the traveling wave generated in the teeth (not shown) side of the stator 432 can be changed by way of controlling the voltage applied to each electrodes of the piezoelectric element array 431. By changing the phase, the teeth (not shown) of the stator 432 rotate the mover 433 forwardly or reversely. In the structure of the ultrasonic linear motor 427, the structure of the known ultrasonic motor can be employed.

### <Control circuit>

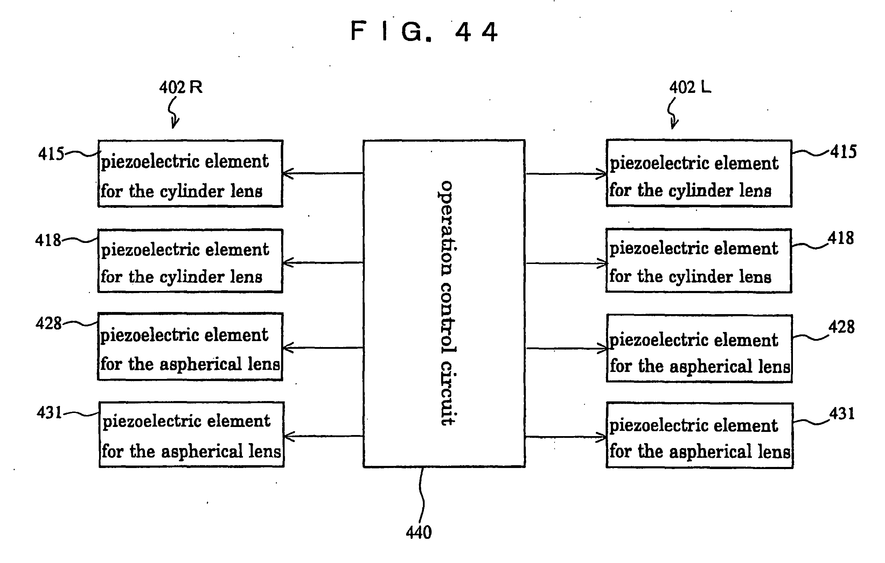

The piezoelectric elements of the piezoelectric element arrays 415, 418, 428, 431 in the above-mentioned lens attaching frames 402R, 402L are controlled to be driven by an operation control circuit 440 shown in FIG. 44 through a communication means (not shown). The communication means may be a wireless communication means or a wire communication means. Also, the operation control circuit 440 is connected with a setting means such as an operation means for operating the setting or change of the spherical degree or the setting of the axis angle of the cylindrical axis, etc. or a data inputting means. As the setting means, a keyboard, a mouse or a button (switch) for data setting can be used. Also, a means for inputting the prescription data of the eyeglasses from the other refract meter or the lens meter may be used as the setting means.

### [Operation]

Next, the operation of the above-mentioned composition will be described.

In order to obtain the final prescription data of the eyeglasses of an test subject 302 which uses the above-mentioned eyeglass frame for test 300a, the test subject 302 wears the eyeglass frame for test 300a as shown in FIG. 37, and while the test subject sees the eye test chart or the astigmatism chart through the variable focus lens 410 (Alvarez lens) and the Vcc lens 423, the above-mentioned variable focus lens 410 (Alvarez lens) or the Vcc lens 423 is operated, and the state that the eye test chart or the astigmatism chart is seen is confirmed.

At this time, in case where the test subject 302 has the measured data of the test subject from the refract meter or has the eyeglasses data of the test subject by the lens meter, the data is inputted to operation control circuit 440 by the communication means, and the operation of the piezoelectric element arrays 415, 418, 428, 431 is controlled by the operation control circuit 440 on the basis of the data as follows.

### (i) Variable operation of spherical degree

The variable operation of the spherical degree by the operation of the variable focus lens 410 (Alvarez lens) can be performed by way of controlling the operation of the piezoelectric element arrays 415, 418.

In this control, the operation control circuit 440 controls the voltage applied to each electrodes of the piezoelectric element arrays 415, 418, and thus, oscillates the teeth (not shown) side of the oscillating body 416 and the teeth (not shown) side of the oscillating body 419 in the inflection standing wave, and generates the traveling wave of the length direction to the teeth (not shown) side of the oscillating body 416 and the teeth (not shown) side of the oscillating body 419. The mover 417 is driven in the upper or lower side by the traveling wave of the teeth (not shown) side of the oscillating body 416, and the mover 420 is driven in the upper or lower side by the traveling wave of the teeth (not shown) side of the oscillating body 419.

At this time, in the operation control circuit 440, the directions of the traveling waves generated in the teeth (not shown) side of the oscillating body 416 and the teeth (not shown) side of the oscillating body 419 are controlled to be the opposite direction each other, and thus, the movers 417, 420 are controlled to be driven (moved) in the opposite directions each other. And, by the vertical movement to the opposite directions of the movers 417, 420, the aspherical lenses 411, 412 supported by the movers 417, 420 are moved upward and downward in the opposite directions each other, and thus, the focuses (spherical refraction degree) by the aspherical lenses 411, 412 are changed. This change is performed on the basis of the measured data of the test subject from the refract meter or the eyeglasses data of the test subject by the lens meter. By changing the focus, the state that the eye test chart is seen by the test subject is confirmed.

Also, in case where there is no measured data of the test subject from the refract meter or the eyeglasses data of the test subject by the lens meter, the operation of the piezoelectric element arrays 415, 418 are controlled by the operation control circuit 440 using the setting means such as the keyboard or the mouse or the button (switch) for data setting from the beginning, the aspherical lenses 411, 412 are moved upward and downward in the opposite direction each other, thereby the focuses by the aspherical lenses 411, 412 are changed (the spherical refraction degree is changed by 0.25D) to obtain the prescription data of the eyeglasses.

And, in case of desiring to change again the state the chart is seen at the spherical degree obtained on the basis of the prescription data or in case where there is a problem at the seen state, that the chart is seen the operation of the piezoelectric element arrays 415, 418 is controlled by the operation control circuit 440 using the setting means such as the keyboard or the mouse or the button (switch) for data setting, the aspherical lenses 411, 412 are moved upward and downward in the opposite directions each other, thereby the focuses (spherical refraction degree) by the aspherical lenses 411, 412 is changed, and the state that the eye test chart is seen by the test subject 302 becomes good.

Since the change is performed by the operation control circuit 440, the data according to the change is obtained by the operation control circuit 440 to become the prescription data of the eyeglasses.

### (ii) Setting of the cylindrical degree

Also, in case where the tested eyes of the test subject 302 has the astigmatism, while the test subject 302 sees the astigmatism chart through the variable focus lens 410 (Alvarez lens) and the Vcc lens 423, the operation of the piezoelectric element arrays 428, 431 are controlled, the rotation of the cylinder lenses 424, 425 of the Vcc lens 423 are relatively controlled, thereby the cylindrical degree of the Vcc lens 423 is changed, and the state that the astigmatism chart is seen is confirmed. The change control of the cylindrical degree is performed as the following descriptions on the basis of the measured data of the test subject from the refract meter or the eyeglasses data of the test subject by the lens meter.

In this control, the operation control circuit 440 can control the voltage applied to each electrodes of the piezoelectric element arrays 428, 431 to change the phase of the traveling wave generated at the teeth (not shown) side of the oscillating bodies 429, 432.

By changing the phase, the teeth (not shown) of the oscillating bodies 429, 432 rotate the movers 430, 433 forwardly or reversely, the cylinder lens 424 of the Vcc lens 423 is rotated or reversely rotated integrally with the mover 430, and the cylinder lens 425 is rotated or reversely rotated integrally with the mover 433.

Accordingly, in the operation control circuit 440, the phases of the traveling waves generated in the teeth (not shown) side of the oscillating bodies 429, 432 are controlled to be opposite each other on the basis of the measured data of the test subject from the refract meter or the eyeglasses data of the test subject by the lens meter, the teeth (not shown) of the oscillating bodies 429, 432 rotate the mover 430, 433 in opposite directions each other, thereby the cylinder lenses 424, 425 are rotated in opposite directions each other to change the cylindrical degree.

And, in case of desiring to change again the state the chart is seen at the spherical degree obtained on the basis of the prescription data or in case where there is a problem at the seen state, the operation of the piezoelectric element arrays 428, 431 are controlled by the operation control circuit 440 using the setting means such as the keyboard or the mouse or the button (switch) for data setting, the cylinder lenses 424, 425 are rotated relatively in the opposite directions each other, thereby the cylindrical degree of the Vcc lens 423 using the cylinder lenses 424, 425 is changed (for example, changed by 0.25D), and the state that the astigmatism chart is seen by the test subject 302 becomes good.

And, in case where there is no measured data of the test subject from the refract meter or the eyeglasses data of the test subject by the lens meter, the operations of the piezoelectric element arrays 428, 431 are controlled by the operation control circuit 440 using the setting means such as the keyboard or the mouse or the button (switch) for data setting from the beginning, the cylinder lenses 424, 425 are rotated in the opposite directions each other, thereby the cylindrical degree of the Vcc lens 423 using the cylinder lenses 424, 425 is changed (for example, changed by 0.25D), and the seen state that the astigmatism chart is seen by the test subject 302 becomes good.

In the change of the cylindrical degree, the operation control circuit 440 operates the variable focus lens 410 (Alvarez lens) to be driven as (i) to cancel the change of spherical degree according to the change of cylindrical degree.

Since the change is performed by the operation control circuit 440, the data according to the change is obtained by the operation control circuit 440 to become the prescription data of the eyeglasses.

### (iii) Setting of the cylindrical axis

Also, in case where the tested eyes of the test subject 302 has the astigmatism, while the test subject 302 sees the astigmatism chart through the variable focus lens 410 (Alvarez lens) and the Vcc lens 423, the operation of the piezoelectric element arrays 428, 431 are controlled, the rotation of the Vcc lens 423 is controlled, the direction of the cylindrical axis of the Vcc lens 423 is operated, and the state that the astigmatism chart is seen is confirmed. The direction change of the cylindrical axis is performed by the same operation as the conventional astigmatism inspecting method. The direction change of the cylindrical axis is performed as follows on the basis of the measured data of the test subject from the refract meter or the eyeglass data of the test subject by the lens meter.

In this control, as mentioned in the above, the operation control circuit 440 can control the voltage applied to each electrodes of the piezoelectric element arrays 428, 431 to change the phase of the traveling wave generated at the teeth (not shown) side of the oscillating bodies 429, 432. By changing the phase, the teeth (not shown) of the oscillating bodies 429, 432 rotate the movers 430, 433 forwardly or reversely, the cylinder lens 424 of the Vcc lens 423 is rotated forwardly or reversely integrally with the mover 430, and the cylinder lens 425 is rotated forwardly or reversely integrally with the mover 433.

Accordingly, the operation control circuit 440 synchronizes the phases of the traveling waves generated in the teeth (not shown) side of the oscillating bodies 429, 432 on the basis of the measured data of the test subject from the refract meter or the eyeglasses data of the test subject by the lens meter, and at the same time, the teeth (not shown) of the oscillating bodies 429, 432 synchronously rotates the movers 430, 433 in circumferential direction on the basis of the measured data of the test subject from the refract meter or the eyeglasses data of the test subject by the lens meter, thereby the cylinder lenses 424, 425 are integrally rotated in circumferential direction to change the direction of the cylindrical axis.

And, in case of desiring to change again the seen state at the cylindrical axis obtained on the basis of the prescription data or in case where there is a problem at the seen state, the operations of the piezoelectric element arrays 428, 431 are controlled by the operation control circuit 440 using the setting means such as the keyboard or the mouse or the button (switch) for data setting, the cylinder lenses 424, 425 are synchronously rotated, thereby the directions of the cylindrical axes of the cylinder lenses 424, 425 are changed, and the state that the astigmatism chart is seen by the test subject 302 becomes good.

And, in case where there is no measured data of the test subject from the refract meter or the eyeglasses data of the test subject by the lens meter, the operations of the piezoelectric element arrays 428, 431 are controlled by the operation control circuit 440 using the setting means such as the keyboard or the mouse or the button (switch) for data setting from the beginning, the cylinder lenses 424, 425 are rotated, thereby the state that the astigmatism chart is seen by the test subject 302 becomes good.

Since the change is performed by the operation control circuit 440, the data according to the change is obtained by the operation control circuit 440 to become the prescription data of the eyeglasses.

And, the control by the above-mentioned operation control circuit 440 is performed on the basis of the measured data of the test subject from the refract meter or the eyeglass data of the test subject by the lens meter, but the control may be performed on the basis of the final corrected value data from the subjective eye test apparatus.

Also, in the above-mentioned example, a pair of aspherical lenses 411, 412 of the variable focus lens 410 are driven by the ultrasonic linear motors (ultrasonic motors) 413, 414, and the cylinder lenses 424, 425 of the Vcc lens 423 are driven by the ultrasonic motors 426, 427, but the present invention is not to limited to that. The power of the pulse motor is transmitted to the aspherical lenses 411, 412 or the cylinder lenses 424, 425 by using the gear power transmitting equipment using general pinion, etc., and the aspherical lenses 411, 412 or the cylinder lenses 424, 425 may be controlled to be driven.

As mentioned in the above, in the eye test system according to in the first aspect of the present invention, since an information detecting portion is provided in a eyeglass frame for test, and an inputting means for inputting the information from said information detecting portion to a computer is provided, the information from the information detecting portion of the eyeglass frame can be automatically detected and inputted to the computer. Also, the detected information can be used for preparing the most suitable eyeglass frame fit for the customer. Thereby, the fittest eyeglass frame for the customer can be accomplished.

Also, in case where the information detecting portion is the information detecting portion of the refraction property of a single or a plurality of exchange lenses, the refraction property of the eyeglasses of the final prescription value by the eyeglass frame for test can be automatically detected and inputted to the computer. Also, in case where the information detecting portion has the information of the movable portion of the eyeglass frame for test, the information such as the eyeglass frame size (dimension) can be automatically detected and inputted to the computer. Also, the obtained eyeglass frame size can be used in preparing the most suitable eyeglass frame fit for the customer's facial size. Thereby, the fittest eyeglass frame for the customer can be accomplished.

Also, in the eye test system according to the second aspect of the present invention, in addition to the first aspect of the present invention, since there is provided the composition that said eyeglass frame for test comprises a lens attaching frame to which a plurality of the exchange lens are mounted so as to be overlapped and be attachable and detachable, and said information detecting portion detects refraction property data of the lens attached to said lens attaching frame, the refraction property data of the eyeglasses of the final prescription value by the eyeglass frame for test can be automatically inputted to the computer.

In the eye test system according to the third aspect of the present invention, in addition to the first or second aspect of the present invention, since there is provided the composition that said eyeglass frame for test comprises a movable portion for changing and adjusting the size and contour according to the user's facial size and contour, and said information detecting portion detects the data from movable portion on said eyeglass frame for test, though the customer does not wear the plurality of eyeglass frames at when selecting the eyeglass frame, the fitting eyeglass frame can be finally obtained. As the result, it is sufficient that the eyeglass frame displayed on the monitor by the personal computer or the catalog is selected. Also, since the eyeglass frame information can be stored as record or memory, it can be used when selecting the next eyeglass frame, and thus, it can be reused by stocking the eyeglass frame information once in the opticians as the data. Thereby, since the customer can connect the opticians using the Internet and select and order the eyeglass frame on the monitor by stocking the eyeglass frame information once as the data, the customer does not have to go to opticians when the customer wants to make a new eyeglass frame.

In the eye test system according to the fourth aspect of the present invention, in addition to the third aspect of the present invention, since there is provided the composition that said eyeglass frame for test at least has a head frame extended leftward and rightward and a nosepiece provided in the center of said head frame, and at the same time, a temple which is provided at the both ends of said head frame as the movable portion such that the length thereof can be telescopically adjusted, and said eyeglass frame for test comprises a length detecting means for detecting the expanded or contracted length of the temple as said information detecting portion, the length information of the temple fit for the person wearing the eyeglasses can be detected. Also, the obtained temple length can be used for setting the most suitable temple length according to the customer's facial size.

In the eye test system according to the fifth aspect of the present invention, in addition to the third aspect of the present invention, since there is provided the composition that said eyeglass frame for test has a nosepiece of which the position can be changed and adjusted as the movable portion, and said information detecting portion is provided such that the position of said nosepiece can be detected, the position information of the nosepiece fit for the person wearing the eyeglasses can be detected. Also, the obtained position information of the nosepiece can be used for setting the most suitable position information of the temple fit for the customer's facial size.

In the eye test system according to the sixth aspect of the present invention, in addition to the fifth aspect of the present invention, since there is provided the composition that said nosepiece is attached to said eyeglass frame for test such that the vertical movement thereof can be adjusted, and said information detecting portion is a nosepiece vertical position detecting means for detecting the position in the vertical direction of said nosepiece, the position information in the height direction of the nosepiece fit for the person wearing the eyeglasses can be detected. Also, the obtained position information of the vertical position of the nosepiece can be used for setting the most suitable position in the height direction of the nosepiece fit for the customer's facial size.

In the eye test system according to the seventh aspect of the present invention, in addition to the fifth aspect of the present invention, since there is provided the composition that said nosepiece is attached to said eyeglass frame for test such that the position thereof can be adjusted forwardly and backwardly, and said information detecting portion is a forward and backward position detecting means for detecting the position of the forward and backward direction of said nosepiece, the position information in the forward and backward direction of the nosepiece fit for the person wearing the eyeglasses can be detected. Also, the obtained position information in the forward and backward direction of the nosepiece can be used for setting the most suitable position in the forward and backward direction of the nosepiece fit for the customer's facial size.

In the eye test system according to the eighth aspect of the present invention, in addition to the fourth aspect of the present invention, since there is provided the composition that a plurality of eyeglass frames for test of which the protruded amount protruded from said head frame are different from each other are prepared, the eyeglass frame for test having the most suitable height to the nosepiece can be simply selected.

In the eye test system according to the ninth aspect of the present invention, in addition to the third aspect of the present invention, since there is provided the composition that said eyeglass frame for test comprises a head frame extended leftward and rightward direction, a nosepiece provided in the center of said head frame and a temple attached to the both ends of said head frame, and at the same time, has a lens attaching frame supported by the left and right portions of said head frame so as to reciprocate in the direction that the head frame is extended is provided as the movable portion, and at the same time, said information detecting portion is a moved-position detecting means for detecting the moved position in the left and right direction of said lens attaching frame, the eye test system can be used for detecting the inter-pupil distance. Also, by this composition, though the person wearing the eyeglasses has severe heterophoria, the most suitable inter-pupil distance can be detected.

In the eye test system according to the tenth aspect of the present invention, in addition to the ninth aspect of the present invention, since there is provided the composition that said lens attaching frame is provided such that movement thereof can be adjusted leftward and rightward by a feed screw supported by said head frame, and said feed screw is controlled to be rotated by a driving motor attached to said head frame, the position of the lens attaching frame can be set simply and quickly at the most suitable position fit for the eyeglass frame user.

In the eye test system according to the eleventh aspect of the present invention, in addition to the ninth aspect of the present invention, since there is provided the composition that said driving motor is driven so as to be controlled by an operation control circuit using an inter-pupil distance data of an test subject, and at the same time, said operation control circuit controls the operation of said driving motor and controls the movement of said lens attaching frame leftward and rightward such that the distance between the centers of said left and right lens attaching frames becomes said inter-pupil distance, in case where the inter-pupil distance data exists in the eye test data of the eyeglass frame for test user, the position of the lens attaching frame can be automatically set simply and quickly at the most suitable position fit for the eyeglass frame for test user using the inter-pupil distance data.

## Claims

1. An eyeglass frame comprising:
a head frame (400) extending in the left and right directions;
a nosepiece (406) provided in the center of said head frame;
temples (20) attached to the both ends of said head frame;
lens attached frames (402R, 402L) respectively provided in the left and right portions of said head frame;
a variable focus lens (410), which is attached to lens attached frame (402R, 402L) said each and is provided to be capable of adjusting the focus;
a cylindrical lens (424, 425) which is attached to said each lens attached frame (402R, 402L),
and is used in combination with said variable focus lens (410), **characterized in that**:
said variable focus lens comprising a pair of aspherical lenses (411, 412) and the focus of the variable focus lens is adjustable by relatively moving said pair of the aspherical lenses (411, 412);
said cylindrical lens (424, 425) is capable of being rotated;
the eyeglass frame further comprises a first motor (413, 414) for driving the pair of the aspherical lenses relatively to each other, a second motor (426, 427) for driving the cylindrical lens, and communicating means (440); and
the first and second motors (413, 414, 426, 427) are driven and controlled via the communicating means based on driving control data, thereby obtaining prescription data of the eyeglasses.

2. The eyeglass frame set forth in claim 1, wherein the driving control data are driving control data based on measured data of eyes of a test subject, measured data of eyeglasses of the test subject, set data of a spherical degree, a cylindrical degree and/or an angle of a cylindrical axis of each of the eyeglasses of the test subject.

3. The eyeglass frame set forth in claim 2, wherein said driving control data are driving control data which are outputted by an operation control circuit (440), separately provided from the eyeglass frame, based on data, through receiving the data, including the measured data of the eyes of the test subject, the measured data of the eyeglasses of the test subject, and/or the set data of the spherical degree, the cylindrical degree and/or the angle of the cylindrical axis of the each of the eyeglasses of the test subject, the operation control circuit (440) being adapted to drive and control the first motor and/or the second motor via the communicating means, and thus the prescription data of the eyeglasses being offered to the test subject at least with respect to the spherical degree, the cylindrical degree and the angle of the cylindrical axis.

4. The eyeglass frame set forth in any one of claims 1 to 3, wherein the lens attached frames are attached to the head frame (400) so as to be adjustable leftwardly and rightwardly.

5. The eyeglass frame set forth in any one of claims 1 to 4, wherein the focus of each of the variable focus lenses is adjustable by vertically moving the pair of the aspherical lenses relatively to each other.

6. The eyeglass frame set forth in any one of claims I to 5, wherein said variable focus lens includes an Alvarez lens.

7. The eyeglass frame set forth in any one of claims 1 to 7, wherein each of the cylindrical lenses includes a variable cross cylinder lens (423).

8. An eye test system comprising the eyeglass test frame defined in any one of claims 1 to 7 and the operation control circuit (440) of claim 3, said operation control circuit being configured to receive the measured data of the eyes of the test subject from a refractometer and/or the data of the eyeglasses of the test subject from a lens meter and then control the first and/or second motor.

9. An eye test system comprising the eyeglass test frame defined in any one of clams 1 to 7, the operation control circuit (440) of claim 3, and a setting means for setting data of the eyeglasses of the test subject, said operation control circuit being configured to receive the set data and then controlling the first and/or second motor.

10. The eye test system set forth in claim 9, wherein said data are the measured data of the eyes of the test subject from the refractometer and/or the data of the eyeglasses from the lens meter, and the operation control circuit being configured to receive the set data by the setting means.

## Patentansprüche

1. Brillenfassung umfassend:
ein Stirngestell (400), das sich in die linke und rechte Richtung erstreckt;
ein Nasenteil (406), das in der Mitte des Stirngestells vorgesehen ist;
Bügel (20), die an beiden Enden des Stirngestells befestigt sind;
Rahmen (402R, 402L) für Befestigung von Brillengläsern, die jeweils in den linken und rechten Bereichen des Stirngestells vorgesehen sind,
ein Brillenglas mit variablem Fokus (410), das an jedem Rahmen (402R, 402L) zur Befestigung von Brillengläsern befestigt ist und vorgesehen ist, in der Lage zu sein, den Fokus einzustellen;
ein Zylinderglas (424, 245), das an jedem Rahmen (402R, 402L) zur Befestigung von Brillengläsern befestigt ist und in Kombination mit dem Brillenglas mit variablem Fokus (410) verwendet wird, **dadurch gekennzeichnet, dass**:
das Brillenglas mit variablem Fokus ein Paar von asphärischen Linsen (411, 412) umfasst und der Fokus des Brillenglases mit variablem Fokus durch relatives Bewegen des Paares von asphärischen Linsen (411, 412) einstellbar ist;
das Zylinderglas (424, 425)in der Lage ist, gedreht zu werden;
die Brillenfassung weiterhin einen ersten Motor (413, 414) zum Antreiben des Paars von asphärischen Linsen relativ zueinander, einen zweiten Motor (426, 427) zum Antreiben des Zylinderglases und Kommunikationsmittel (440) umfasst; und
der erste und zweite Motor (413, 414, 426, 427) über die Kommunikationsmittel auf der Grundlage von Antriebssteuerdaten angetrieben werden, wodurch Verordnungsdaten für die Brille erhalten werden.

2. Brillenfassung nach Anspruch 1, wobei die Antriebssteuerdaten solche sind, die auf gemessenen Daten von Augen einer Testperson, gemessenen Daten von Brillengläsern der Testperson, gesetzten Daten eines sphärischen Wertes, eines zylindrischen Wertes und/oder eines Winkels einer zylindrischen Achse jedes der Brillengläser der Testperson basieren.

3. Brillenfassung nach Anspruch 2, **dadurch gekennzeichnet, dass** die Antriebssteuerdaten solche sind, die von einem Operationssteuerkreis (440) ausgegeben werden, der getrennt von der Brillenfassung vorgesehen ist, basierend auf Daten, durch das Empfangen der Daten, einschließlich der gemessenen Daten der Augen der Testperson, der gemessenen Daten der Brillengläser der Testperson und/oder der gesetzten Daten des sphärischen Wertes, des zylindrischen Wertes und/oder des Winkels der zylindrischen Achse jedes der Brillengläser der Testperson, wobei der Operationssteuerkreis (440) geeignet ist, den ersten Motor und/oder den zweiten Motor über die Kommunikationsmittel anzutreiben und zu steuern und wobei somit die Verordnungsdaten der Brillengläser der Testperson mindestens in Bezug auf den sphärischen Wert, den zylindrischen Wert und den Winkel der Zylinderachse angeboten wird.

4. Brillenfassung nach einem der Ansprüche 1 bis 3, wobei die Rahmen zur Befestigung von Brillengläsern an dem Stirngestell (400) derart befestigt sind, dass sie nach links und nach rechts einstellbar sind.

5. Brillenfassung nach einem der Ansprüche 1 bis 4, wobei der Fokus jeder der Linsen mit variablem Fokus durch vertikales Bewegen des Paares von asphärischen Linsen relativ zueinander einstellbar ist.

6. Brillenfassung nach einem der Ansprüche 1 bis 5, wobei die Linse mit variablem Fokus eine Alvarez-Linse einschließt.

7. Brillenfassung nach einem der Ansprüche 1 bis 6, wobei jedes der Zylindergläser ein variables Kreuzzylinderglas (423) einschließt.

8. Augentestsystem, das Brillentestgestell nach einem der Ansprüche 1 bis 7 und den Operationssteuerkreis (440) nach Anspruch 3 umfassend, wobei der Operationssteuerkreis ausgebildet ist, die gemessenen Daten der Augen der Testperson von einem Refraktometer und/oder die Daten der Brillengläser der Testperson von einem Linsenmesser zu empfangen und dann den ersten und/oder den zweiten Motor zu steuern.

9. Augentestsystem, das Brillentestgestell nach einem der Ansprüche 1 bis 7 und den Operationssteuerkreis (440) nach Anspruch 3 und Setzmittel zum Setzen von Daten der Brillengläser der Testperson umfassend, wobei der Operationssteuerkreis ausgebildet ist, die gesetzten Daten zu empfangen und dann den ersten und/oder zweiten Motor zu steuern.

10. Augentestsystem nach Anspruch 9, wobei die Daten die gemessenen Daten der Augen der Testperson von einem Refraktometer und/oder die Daten der Brillengläser von dem Linsenmesser sind und der Operationssteuerkreis ausgebildet ist, die gesetzten Daten von den Setzmitteln zu empfangen.

## Revendications

1. Monture de lunettes, comprenant :
une face (400) s'étendant dans les sens gauche et droit ;
un pont (406) positionné au centre de ladite face ;
des branches (20) fixées aux deux extrémités de ladite face ;
des montures de fixation de verres (402R, 402L) respectivement positionnées dans les parties gauche et droite de ladite face ;
un verre progressif (410) qui est fixé à chacune desdites montures de fixation de verres (402R, 402L) est fourni, capable d'ajuster le foyer ; et
un verre cylindrique (424, 425) qui est fixée à chacune desdites montures de fixation de verres (402R, 402L) et qui est utilisée en association avec ledit verre progressif (410), **caractérisée en ce que** :
ledit verre progressif comprend une paire de verres asphériques (411, 412) et le foyer du verre progressif est réglable par déplacement relatif de ladite paire de verres asphériques (411, 412),
ledit verre cylindrique (424, 425) peut être entraîné en rotation ;
la monture de lunettes comprend en outre un premier moteur (413, 414) pour entraîner la paire des verres asphériques l'un par rapport à l'autre, un second moteur (426, 427) pour entraîner le verre cylindrique, et des moyens de communication (440) ; et
le premier et le second moteur (413, 414, 426, 427) sont entraînés et commandés via les moyens de communication d'après des données de commande d'entraînement, obtenant ainsi des données de prescription des lunettes.

2. Monture de lunettes selon la revendication 1, dans laquelle les données de commande d'entraînement sont des données de commande d'entraînement d'après des données mesurées des yeux d'un sujet examiné, des données mesurées de lunettes du sujet examiné, des données établies d'un degré de sphéricité, un degré de cylindricité et/ou un angle d'un axe cylindrique de chacune des lunettes du sujet examiné.

3. Monture de linettes selon la revendication 2, dans laquelle lesdites données de commande d'entraînement sont des données de commande d'entraînement qui sont sorties par un circuit de commande de fonctionnement (440), fournies distinctement de la monture de lunettes, d'après des données, par réception des données, comprenant les données mesurées des yeux du sujet examiné, les données mesurées des lunettes du sujet examiné, et/ou les données établies du degré de sphéricité, du degré de cylindricité et/ou de l'angle de l'axe cylindrique de chacune des lunettes du sujet examiné, le circuit de commande de fonctionnement (440) étant adapté pour entraîner et commander le premier moteur et/ou le second monteur via les moyens de communication, et ainsi les données de prescription des lunettes sont proposées au sujet examiné au moins par rapport au degré de sphéricité, au degré de cylindricité et à l'angle de l'axe cylindrique.

4. Monture de lunettes selon l'une quelconque des revendications 1 à 3, dans laquelle les montures de fixation de verres sont fixées à la face (400) de sorte à être réglables vers la gauche et vers la droite.

5. Monture de lunettes selon l'une quelconque des revendications 1 à 4, dans laquelle le foyer de chacun des verres progressifs est réglable en déplaçant verticalement la paire des lentilles asphériques l'une par rapport à l'autre.

6. Monture de lunettes selon l'une quelconque des revendications 1 à 5, dans laquelle ledit verre progressif comprend un verre Alvarez.

7. Monture de lunettes selon l'une quelconque des revendications 1 à 6, dans laquelle chacun des verres progressifs comprend un verre variable à cylindre croisé (423).

8. Système pour l'examen des yeux comprenant la monture de lunettes d'examen définie selon l'une quelconque des revendications 1 à 7 et le circuit de commande de fonctionnement (440) selon la revendication 3, ledit circuit de commande de fonctionnement étant configuré pour recevoir les données mesurées des yeux du sujet examiné à partir d'un réfractomètre et/ou des données des lunettes du sujet examiné à partir d'un frontofocomètre et commander ensuite le premier et/ou le second moteur.

9. Système pour l'examen des yeux comprenant la monture de lunettes d'examen selon l'une quelconque des revendications 1 à 7, le circuit de commande de fonctionnement (440) selon la revendication 3, et des moyens de réglage pour établir les données des lunettes du sujet examiné, ledit circuit de commande de fonctionnement étant configuré pour recevoir les données établies et pour commander ensuite le premier et/ou le second moteur.

10. Système pour l'examen des yeux selon la revendication 9, dans lequel lesdites donnés sont les données mesurées des yeux du sujet examiné provenant du réfractomètre et/ou les données des lunettes du sujet examiné provenant du frontofocomètre, et le circuit de commande de fonctionnement est configuré pour recevoir les données établies par les moyens de réglage.
